# EUROPEAN PATENT SPECIFICATION

(11) **EP 1 976 507 B1**
(45) Date of publication and mention of the grant of the patent: **05.09.2012**
(21) Application number: 06847861.9
(22) Date of filing: 19.12.2006
(51) Int. Cl.: A61K 31/353, A61K 31/357, A61P 25/08

(54) **USE OF BENZO-FUSED HETEROCYCLE SULFAMIDE DERIVATIVES FOR THE TREATMENT OF EPILEPTOGENESIS**
VERWENDUNG VON BENZOKONDENSIERTEN HETEROCYCLISCHEN SULFAMID-DERIVATEN ZUR BEHANDLUNG VON EPILEPTOGENESE
UTILISATION DE DERIVES DE SULFAMIDE HETEROCYCLIQUE BENZO-FUSIONNE POUR TRAITER L'ÉPILEPTGENÈSE

(30) Priority: 19.12.2005 US 751496 P; 18.12.2006 US 612174
(43) Date of publication of application: 08.10.2008
(73) Proprietor: Janssen Pharmaceutica N.V., 2340 Beerse (BE)
(72) Inventor: SMITH-SWINTOSKY, Virginia L., Hatfield, Pennsylvania 19440 (US)
(74) Representative: Warner, James Alexander
(86) International application number: PCT/US2006/048682
(87) International publication number: WO 2007/075834

(56) References cited:
- WO-A-2006/007435
- WO-A-2006/007436
- MARYANOFF B E ET AL: "Comparison of Sulfamate and Sulfamide Groups for the Inhibition of Carbonic Anhydrase-II by Using Topiramate as a Structural Platform" JOURNAL OF MEDICINAL CHEMISTRY, AMERICAN CHEMICAL SOCIETY. WASHINGTON, US, vol. 48, no. 6, 13 December 2004 (2004-12-13), pages 1941-1947, XP002345002 ISSN: 0022-2623
- WAUGH J ET AL: "Topiramate: As Monotherapy in Newly Diagnosed Epilepsy" CNS DRUGS 2003 NEW ZEALAND, vol. 17, no. 13, 2003, pages 985-992, XP009083685 ISSN: 1172-7047
- GUILLAUME D ET AL: "Glial contribution to seizure: Carbonic anhydrase activity in epileptic mammalian brain" EPILEPSIA 1991 UNITED STATES, vol. 32, no. 1, 1991, pages 10-15, XP009083684 ISSN: 0013-9580

## Description

### FIELD OF THE INVENTION

The present invention is relates methods for the use of benzo-fused heterocycle sulfamide derivatives to therapeutically or prophylactically treat, prevent, reverse, arrest or inhibit epileptogenesis,

### BACKGROUND OF THE INVENTION

Injuries or trauma of various kinds to the central nervous system (CNS) or the peripheral nervous system (PNS) can produce profound and long-lasting neurological and psychiatric symptoms and disorders. One common mechanism for the production of these effects is the induction of seizure activity or seizure-like phenomena in the CNS or in the nerves and ganglia of the PNS. Symptomatic of paroxysmal disturbances in CNS or PNS electrical activity, seizures or seizure-like neurological mechanisms are believed to underlie many of the pathological phenomena in a wide variety of neurological and psychiatric disorders.
One serious neurological condition characterized by seizures is epilepsy. Epilepsy is a common but devastating disorder affecting more than two and a half million people in the United States alone. Epilepsy describes a condition in which a person has recurrent seizures due to a chronic, underlying process. Epilepsy refers to a clinical phenomenon rather than a single disease entity, since there are many forms and causes of epilepsy. Using a definition of epilepsy as two or more unprovoked seizures, the incidence of epilepsy is estimated at approximately 0.3 to 0.5 percent in different populations throughout the world, with the prevalence of epilepsy estimated at 5 to 10 people per 1000.
On the basis of clinical and encephalographic phenomenon, four subdivisions of epilepsy are recognized: grand mal epilepsy (with subgroups: generalized, focal, jacksonian), petit mal epilepsy, psychomotor or temporal lobe epilepsy (with subgroups: psychomotor proper or tonic with adversive or torsion movements or masticatory phenomenon, automatic with amnesia, or sensory with hallucinations or dream states) and autonomic or diencephalic epilepsy (with flushing, pallor, tachycardia, hypertension, perspiration or other visceral symptoms).
While epilepsy is one of the foremost examples of a seizure-related disorder, a wide variety of neurological and psychiatric symptoms and disorders may have, as their etiology, seizures or related seizure-like neurological phenomenon. In simple terms, a seizure or a related seizure-like neurological phenomenon is a single discrete clinical event caused by an excessive electrical discharge from a collection of neurons or a seizure susceptible group of neurons through a process termed "ictogenesis." As such, ictogenic seizures may be merely the symptom of a disease. However, epilepsy and other analogous seizure-related disorders are dynamic and often progressive diseases, with a maturation process characterized by a complex and poorly understood sequence of pathological transformations.
The development and maturation of such changes is the process of "epileptogenesis," whereby the larger collection of neurons that is the normal brain is altered and subsequently becomes susceptible to abnormal, spontaneous, sudden, recurrent, excessive electrical discharges, i.e., seizures. The maturation of the epileptogenic process results in the development of an "epileptogenic focus," whereby the collections of abnormally discharging neurons or neurons susceptible to seizures form localized groups or "epileptogenic zones" interspersed throughout the cortical tissue. The epileptogenic zones are biochemically inter-connected such that an abnormal ictogenic discharge is able to cascade from zone to zone.

As epileptogenesis progresses, the involved areas of the nervous system become more susceptible to a seizure and it becomes easier for a seizure to be triggered, resulting in progressively debilitating symptoms of the seizure or seizure-related disorder.
While ictogenesis and epileptogenesis may have a common origin in certain biochemical phenomenon and common neuronal pathways in various diseases, the two processes are not identical. Ictogenesis is the initiation and propagation of a seizure in a discrete time and space, a rapid and definitive electrical/chemical event that occurs over a period of time ranging from seconds to minutes.
Comparatively, epileptogenesis is a gradual biochemical or neuronal restructuring process whereby the normal brain is transformed by ictogenic events into an epileptogenically focused brain, having neuronal circuitry that becomes sensitized and responsive to ictogenic events, making an individual increasingly susceptible to the recurrence of spontaneous, episodic, time-limited seizures, resulting in progressively debilitating symptoms of the seizure or seizure-related disorder and progressive non-responsiveness to treatment. The maturation of an "epileptogenic focus" is a slow biochemical and/or structural process that generally occur over months to years.
Epileptogenesis is a Two Phase Process: "Phase 1 epileptogenesis" is the initiation of the epiteptogenic process prior to the first epileptic seizure or symptom of an analogous seizure-related disorder, and is often the result of some kind of injury or trauma to the brain, i.e., stroke, disease (e.g., infection such as meningitis), or trauma; such as an accidental blow to the head or a surgical procedure performed on the brain. "Phase 2 epileptogenesis" refers to the process during which brain tissue that is already susceptible to epileptic seizures or seizure related phenomena of an analogous seizure-related disorder, becomes still more susceptible to seizures of increasing frequency and/or severity and/or becomes less responsive to treatment.
While the processes involved in epileptogenesis have not been definitively identified, some researchers believe that the up regulation of excitatory coupling between neurons, mediated by N-methyl-D-aspartate (NMDA) receptors, is involved. Other researchers implicate down regulation of inhibitory coupling between neurons, mediated by gamma-amino-butyric acid (GABA) receptors. Many other factors may be involved in this process relating to the presence, concentration or activity of NO (nitric oxide) or iron, calcium or zinc ions.
Although epileptic seizures are rarely fatal, large numbers of patients require medication to avoid the disruptive, and potentially dangerous consequences of seizures. In many cases, medication used to manage the epileptic seizures or symptoms of an analogous seizure-related disorder is required for extended periods of time, and in some cases, a patient must continue to take such prescription medication for life. Furthermore, such drugs are only effective for the management of symptoms and have side effects associated with chronic, prolonged usage.
A wide variety of drugs available for the management of epileptic seizures include older agents such as phenytoin, valproate and carbamazepine (ion channel blockers), as well as newer agents such as felbamate, gabapentin, topiramate and tiagabine. In addition, for example, β-alanine has been reported to have anti-seizure activity, NMDA inhibitory activity and GABAergic stimulatory activity, but has not been employed clinically to treat epilepsy.
Accepted drugs for the treatment of epilepsy are anticonvulsant agents or, more properly termed, anti-epileptic drugs (AEDs), wherein the term "anti-epileptic" is synonymous with "anti-seizure" or "anti-ictogenic". These drugs therapeutically suppress seizures by blocking the initiation of a single ictogenic. event. But those AED's now clinically available, do not prevent the process of epileptogenesis.
In treating seizures or related symptoms of analogous seizure-related disorders, that is for diseases and disorders with seizure-like neurological phenomenon that may apparently be related to seizures disorders, such as mood cycling in Bipolar Disorder, impulsive behavior in patients with Impulse Control Disorders or for seizures resulting from brain injury, some AEDs may also be therapeutically useful. However, those AED's now approved are unable to prophylactically or therapeutically prevent the initial development or progressive maturation of epileptogenesis to an epileptogenic focus that also characterizes analogous seizure-related disorders.

The poorly understood pathological mechanisms that underlie epileptogenesis certainly play a role in the development of epilepsy and analogous seizure-related disorders under a variety of clinical circumstances including spontaneous development or as a result of injury or trauma of many kinds to the central or peripheral nervous system.
Current epilepsy treatment is focused on suppressing seizure activity by administering AEDs after overt clinical epilepsy has developed. Although AEDs have positive effects in suppressing seizures, those now available have been universally unsuccessful in preventing epileptogenesis, i.e., the initial development or progression and worsening of epilepsy and other related seizure-like diseases. Even pretreatment with AEDs does not prevent the development of epilepsy after injury or trauma to the nervous system. Moreover, if therapy with AEDs is discontinued, the seizures typically recur and, in unfortunate instances, worsen with time. Currently, there is no clinically available method for treating, preventing, reversing, arresting or inhibiting the onset and/or progression of epilepsy or other seizure disorders or the many analogous seizure-related disorders.
In addition, it is also believed that similar neurological mechanisms corresponding to epileptogenesis may be involved in the evolution and development of many seizure-related disorders clinically analogous to epilepsy that do not appear to be overtly "epileptic," such as the initial development and progressive worsening observed in the mature disease state in Bipolar Disorder, Impulse Control Disorders, Obsessive-Compulsive disorders, Schizoaffective disorders, Substance Abuse or Addictive Disorders and many other psychiatric and neurological disorders.
Thus, despite the numerous drugs available for the treatment of epilepsy (i.e., through suppression of ictus epilepticus, i.e., the convulsions associated with epileptic seizures) and other analogous seizure-related disorders, there are no generally accepted drugs for treating, preventing, reversing, arresting or inhibiting the underlying process of epileptogenesis that may be etiologic in many devastating neurological and psychiatric disorders such as epilepsy and analogous seizure-related disorders including Bipolar Disorder.

Currently, there are no known methods of inhibiting the epileptogenic process to prevent the development of epilepsy or other analogous seizure-related disorders in patients who have not yet clinically shown symptoms thereof, but who unknowingly have the disease or are at risk of developing the disease. In addition, there are no known methods to prevent the development of or reverse the process of epileptogenesis, thus converting the collections of neurons in an epileptogenic zone which have been the source of or are susceptible or are capable of participating in seizure activity into nerve tissue that does not exhibit abnormal, spontaneous, sudden, recurrent or excessive electrical discharges or is not susceptible to or capable of such seizure activity. Furthermore, there are no approved or unapproved medications recognized as having such anti-epileptogenic properties, i.e., truly anti-epileptogenic drugs (AEGDs) (See, Schmidt, D. and Rogawski, M. A., Epilepsy Research, 2002, 50; 71-78) .
WO 2006/007436 and WO 2006/007435 both disclose the use of the presently claimed compounds in the treatment of epilepsy.
Thus, there is a great need to develop safe and effective drugs or AEDs and methods of treatment that effectively, treat, prevent, arrest, inhibit and / or reverse epileptogenesis in seizure-related neurological and/or psychiatric disorders, preferably, in addition to suppressing the seizures or convulsions or seizure related symptoms in patients who already manifest these types of symptoms.

### SUMMARY OF THE INVENTION

The present invention relates to a method for the treatment, prevention, arrest, inhibition and / or reversal of epileptogenesis comprising administering to a subject in need thereof a therapeutically effective amount of a compound of formula (I) 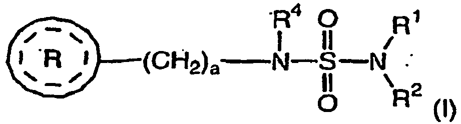 wherein
R¹ and R² are each independently selected from the group consisting of hydrogen and lower alkyl;
R⁴ is selected from the group consisting of hydrogen and lower alkyl;
a is an integer from 1 to 2; 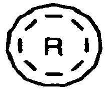 is selected from the group consisting of 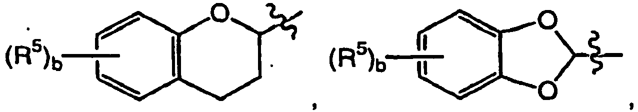 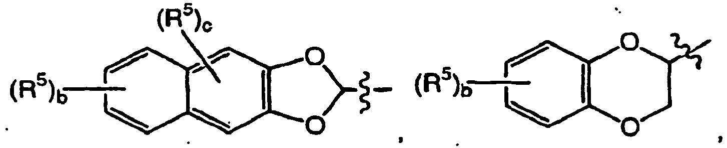 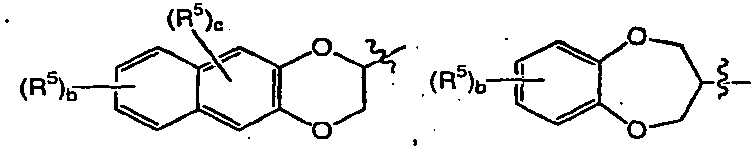 and 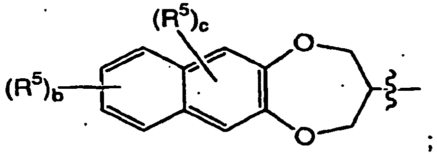 wherein b is an integer from 0 to 4; and wherein c is an integer from 0 to 2;
   each R⁵ is independently selected from the group consisting of halogen, lower alkyl and nitro;
   provided that when 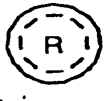 is 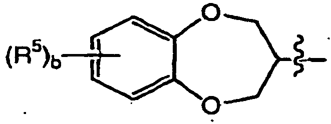 or 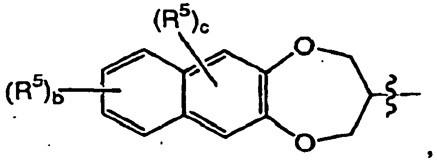 then a is 1;
   or a pharmaceutically acceptable salt thereof.

The present invention further relates to a method for the treatment, prevention, arrest, inhibition and / or reversal of epileptogenesis comprising administering to a subject in need thereof a therapeutically effective amount of compound of formula (II) 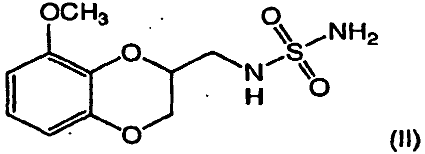 or a pharmaceutically acceptable salt thereof.

In an embodiment, the present invention relates to methods of treatment, prevention, arrest, inhibition and/or reversal of epileptogenesis in seizure-related neurological and / or psychiatric disorders comprising administering to a subject in need thereof a therapeutically effective amount of a compound of formula (I) and / or formula (II) as described herein.

In another embodiment, the present invention relates to methods for the treatment, prevention, arrest, inhibition and / or reversal of epileptogenesis in a patient at risk for the development of a epilepsy, seizure disorder or an analogous seizure-related disorder.
In another embodiment, the present invention relates to an improved method for treating and preventing seizures and seizure-related disorders in a subject in need thereof. This method includes the step of prophylactically administering to the subject in need thereof a therapeutically effective amount of any of the compounds described herein that prevents the occurrence of seizures, convulsions or seizure-related disorders in the subject while simultaneously suppressing epileptogenesis.

In embodiments of the present invention, a prophytactically effective amount of a pharmaceutical composition for preventing seizures or convulsions or seizure-related disorders in patients who have already shown symptoms of such disorders, comprising one or more of the compounds of formula (I) and / or formula (II), in admixture with a pharmaceutically acceptable carrier or excipient, is administered to the subject in need of such treatment.
In additional embodiments, a prophylactically or therapeutically effective amount of a pharmaceutical composition for preventing, treating, reversing, arresting and / or inhibiting epileptogenesis comprising one or more of the compounds of formula (I) or formula (II) in admixture with a pharmaceutically acceptable carrier or excipient, whereby such a composition is administered to the subject in need of treatment with an AEGD. Pharmaceutical compositions comprising at least one compound of formula (I) and / or formula (II) and one or more pharmaceutically acceptably excipients are administered to a subject in need thereof.

The present invention further relates to methods for the treatment, prevention, arrest, inhibition and / or reversal of epileptogenesis comprising co-therapy with a therapeutically effective amount of at least one suitable pharmaceutical agent and at least of the compounds of formula (I) and / or formula (II) as described herein.

### DETAILED DESCRIPTION OF THE INVENTION

The present invention relates to a method for the treatment and/or prevention of epileptogenesis comprising administering to a subject in need thereof a therapeutically effective amount of a compound of formula (I) 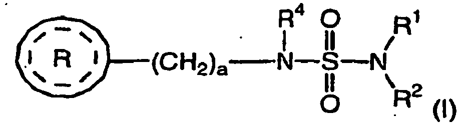 or a pharmaceutically acceptable salt thereof, wherein 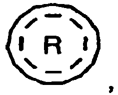 a, R¹, R² and R⁴ are as herein defined.

Compounds of formula (I) and formula (II) are anticonvulsants and can suppress epileptic seizures. In addition, the compounds of formula (I) and formula (II) have been inexpertly found to be are powerfully anti-epileptogenic compounds that can prevent the initial development and maturation of the pathological changes in the nervous system that allow seizures and related phenomena to occur and/or spread. The compounds of formula (I) and formula (II) may further be able to reverse the changes resulting from epileptogenesis. Thus, the compounds of formula (I) and formula (II) of the present invention, as used in the methods of this invention, are true anti-epileptogenic drugs (AEGDs) and have properties that are distinctly different from and not possessed by any presently approved AED medication.

The present invention therefore relates to methods of treating, preventing, reversing, arresting and / or inhibiting epileptogenesis. In certain embodiments, these methods comprise administering a prophylactically or therapeutically effective amount of a compound of formula (I) or formula (II) to the subject in need thereof.
One skilled in the art will recognize that before prophylactic or therapeutic administration of the any of the compounds described herein for the treatment, prevention, inhibition, reversal or arrest of epileptogenesis, a determination will be made as to whether or not the subject suffers from epilepsy or an analogous seizure-related disorder or is considered to be at a high risk for the development of such seizures or seizure-related disorders.

In particular, a subject or patient in need of treatment with an antiepileptogenesis drug (AEGD), may be a subject who has not shown the symptoms of epilepsy or analogous seizure-related disorders, i.e., seizures or convulsions prior to the time of administration.

In another embodiment of the present invention, the subject or patient in need of treatment will be determined to be at risk for developing epilepsy or an analogous seizure-related disorder at the time of administration and on this basis will be considered to be a patient in need of treatment with an AEGD.

In an embodiment, the present invention relates to methods for the treatment, prevention, inhibition, arrest and / or reversal of epileptogenesis, regardless of the initiating and / or underlying cause.
In an embodiment, the present invention relates to methods of treating, preventing, reversing, arresting, and / or inhibiting epileptogenesis comprising administering a therapeutically effective amount of a compound of formula (I) or formula (II) as described herein to a patient who has not developed epilepsy or any type of seizure disorder or an analogous seizure related disorder but who may be in a high risk group for the development of seizures or an analogous seizure related disorder because of injury or trauma to the nervous system that has occurred, including head injury or stroke or may occur in the future, including planned neurosurgical procedures or because of some known predisposition either biochemical or genetic or the finding of a verified biomarker of one or more of these disorders.

In treating epileptogenesis, the compounds of the invention can forestall the development of seizures, particularly epileptic seizures. Such compounds therefore can be used to treat and prevent epilepsy and epileptic seizures, reduce the risk of developing epilepsy, arrest the development of epilepsy (particularly, the development of collections of neurons which are the source of or are susceptible to ictogenic seizure), inhibit the development and maturation of epilepsy (particularly, the development of epileptogenic zones and epileptogenic focus), reduce the severity of epilepsy in a subject and reverse the process of epileptogenesis in epilepsy.
In addition, by treating, preventing, inhibiting, arresting and / or reversing epileptogenesis according to the methods of the present invention, the development or progression of analogous neurological and/or psychiatric disorders whose etiology is partly or wholly based on a seizure like mechanism of action will be treated, prevented, inhibited, arrested and / or reversed.

As used herein, the term "**epileptogenesis**" shall mean the biochemical, genetic, histological or other structural or functional processes or changes that make nervous tissue, including the central nervous system (CNS) susceptible to recurrent, spontaneous seizures. In addition, the term "epileptogenesis" is also used herein in a broader sense to refer to the changes and / or processes that contribute to the clinical progression observed in patients with epilepsy or other seizure disorder or an analogous seizure-related disorder including but not limited to; the worsening or progression of the disorder and it's symptoms or the development of "pharmacoresistance," in which the disorder becomes more difficult to treat as a result of neurobiological changes which result in reduced drug sensitivity or the recruitment by the process of epileptogenesis of non seizure prone nervous tissue.
Furthermore the term "epileptogeriesis" is used herein in the broadest possible sense to refer to the similar phenomena of progressive worsening over time of the signs and symptoms of apparently non-epileptic disorders, including psychiatric disorders the etiology of which appear to be seizure related.

As used herein, the term "**inhibition of epileptogenesis**" refers to preventing, slowing, halting and / or reversing the process of epileptogenesis.
The term "**anti-epileptogenic agent or drug**" and the abbreviation "AEGD", as used herein, refers to an agent that is capable of inhibiting epileptogenesis when the agent is administered to a subject in need thereof.

As used herein the terms "**convulsive disorder**" and "**seizure disorder**" refer to a disorder in which the subject suffers from convulsions, e.g., convulsions due to epileptic seizure. Convulsive disorders include, but are not limited to, epilepsy and non-epileptic convulsions, e.g., convulsions due to administration of a convulsive agent or toxin to the subject.
As used herein, the terms "**analogous seizure-related disorder(s)**" or "**epilepsy related seizure like neurological phenomenon**" refer to a neurobiological disorder or a psychiatric disorder that may show little or no overt seizure activity but which are still believed to be wholly or partly the result of a seizure-like or related neural mechanisms and which are often found to be treatable with AEDs.

As used herein, the term "**subject**" refers to an animal, preferably a mammal, most preferably a human, who has been the object of treatment, observation or experiment. As used herein, the term "subject" or "patient" also includes a subject, preferably a human being, who has not yet shown the symptoms of epilepsy or analogous seizure-related disorder but who may be in a high risk group.

As used herein, the term "**a subject in need of treatment with an AEGD**" includes to any individual with a history of or who currently has; epilepsy, a seizure disorder or an analogous epilepsy related seizure like neurological phenomenon or seizure related disorder, or any disorder in which the patient's present clinical condition or prognosis that could benefit from the suppression or inhibition of the process of epileptogenesis to prevent the extension, progression, worsening or increased resistance to treatment of any neurological or psychiatric disorder.
The term "**a subject in need of treatment with an AEGD**" also includes any individual who does not have epilepsy or and analogous seizure-related disorder but who may be in a high-risk group for the development of seizures or a seizure related disorder because of injury or trauma to the central (CNS) or peripheral nervous system (PNS). An individual or patient is considered to be at a high risk for the development of such seizures or seizure-related disorders because of injury or trauma to the CNS or PNS, because of some known biochemical or genetic predisposition to epilepsy or analogous seizure-related disorder, or because a verified biomarker or surrogate marker of one or more of these disorders has been discovered.
The term "**a subject in need of treatment with an AEGD**" also includes any individual whose clinical condition or prognosis could benefit from treatment with an AEGD. This includes any individual determined to be at an increased risk of developing epilepsy, a seizure disorder or analogous seizure-related disorder or epilepsy related seizure like neurological phenomenon or seizure related disorder as defined above, due to any predisposing factor. Predisposing factors include, but are not limited to: injury or trauma of any kind to the CNS or PNS; infections of the CNS, e.g., meningitis or encephalitis; anoxia; stroke, i.e., cerebro-vascular accidents (CVAs); autoimmune diseases affecting the CNS, e.g., lupus; birth injures, e.g., perinatal asphyxia; cardiac arrest; therapeutic or diagnostic vascular surgical procedures, e.g., carotid endarterectomy or cerebral angiography; heart bypass surgery; spinal cord trauma; hypotension; injury to the CNS from emboli, hyper or hypo perfusion of the CNS; hypoxia affecting the CNS; known genetic predisposition to disorders known to respond to AEGDs; space occupying lesions of the CNS; brain tumors, e.g., glioblastomas; bleeding or hemorrhage in or surrounding the CNS, e.g., intracerebral bleeds or subdural hematomas; brain edema; febrile convulsions; hyperthermia; exposure to toxic or poisonous agents; drug intoxication, e.g. cocaine; family history of seizure disorders or analogous seizure related disorder, history of status epilepticus; current treatment with medications that lower seizure threshold, e.g., lithium carbonate, thorazine or clozapine; evidence from surrogate markers or biomarkers that the patient is in need of treatment with an anti-epileptogenic drug, e.g. MRI scan showing hippocampal sclerosis or other CNS pathology, elevated serum levels of neuronal degradation products.

As used herein, unless otherwise noted, the term "**epilepsy**" shall mean any disorder in which a subject (preferably a human adult, child or infant) experiences one or more seizures and/or tremors. Suitable examples include, but are not limited to, epilepsy (including, but not limited to, localization-related epilepsies, generalized epilepsies, epilepsies with both generalized and local seizures, and the like), seizures as a complication of a disease or condition (such as seizures associated with encephalopathy, phenylketonuria, juvenile Gaucher's disease, Lundborg's progressive myoclonic epilepsy, stroke, head trauma, stress, hormonal changes, drug use or withdrawal, alcohol use or withdrawal, sleep deprivation, and the like) and the like. The term is intended to refer to the clinical disorder regardless of type of seizure, origin of seizure, progression of seizure or underlying cause or etiology.

The term "**antiepileptic drug**" and the abbreviation "**AED**" will be used interchangeably with the term "**anticonvulsant agent**," and as used herein, refer to an agent capable of; treating, inhibiting or preventing seizure activity or ictogenesis when the agent is administered to a subject or patient.
As used herein, the term "**a subject in need of treatment with an AED**" includes any individual who is known to suffer from epilepsy or who has had repeated seizures or convulsions or has shown the symptoms of an analogous seizure-related disorder regardless of the etiology of these symptoms.

As used herein, the terms "**treating**" or "**treatment**", refer to actions that cause any indicia of success in the prevention or amelioration of an injury, pathology, symptoms or condition, including any objective or subjective parameters such as abatement; remission; diminishing of symptoms or making the injury, pathology, or condition more tolerable to the patient; slowing in the rate of degeneration or decline; making the final point of degeneration less debilitating; or improving a subject's physical or mental well-being.
Thus the terms "**treatment**" or "to **treat**" include any action that improves, prevents, reverses, arrests, and / or inhibits the pathological process of epileptogenesis, as that term is defined and used herein. The treatment, or amelioration of symptoms can be based on objective or subjective parameters; including the results of a physical examination, neurological examination, and/or psychiatric evaluations. Accordingly, the term "**treating**" or "**treatment**" includes the administration of the compounds or agents of the present invention to treat, prevent, reverse, arrest, and / or inhibit the process of epileptogenesis. In some instances, treatment with the compounds of the present invention will prevent, inhibit, and / or arrest the progression of brain dysfunction or brain hyperexcitability associated with epilepsy.

As used herein, the term "**therapeutic effect**" refers to the treatment, inhibition, abatement, reversal, and / or prevention of epileptogenesis, the effects or symptoms of epileptogenesis, or side effects of epileptogenesis in a subject.
The term "**therapeutically effective amount**" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated. Thus the terms "**therapeutically effective amount**" or "**therapeutically effective dose**" which are used interchangeably herein, mean a sufficient amount or dose of one or more of the compounds or compositions of the invention to produce a therapeutic effect, as defined above, in a subject or patient in need of such; treatment, inhibition, abatement, reversal, and / or prevention of epiteptogenesis, the effects or "symptoms of epileptogenesis, or side effects of epileptogenesis. The range of doses required for these different therapeutic effects will differ according to the characteristics of the subject or patient and the precise nature of the condition being treated.
Wherein the present invention is directed to co-therapy or combination therapy, comprising administration of one or more compound(s) of formula (I) or formula (II) and one or more "suitable pharmaceutical agent", "therapeutically effective amount" shall mean that amount of the combination of agents taken together so that the combined effect elicits the desired biological or medicinal response. For example, the therapeutically effective amount of co-therapy comprising administration of a compound of formula (I) or formula (II) and at least one suitable pharmaceutical agent would be the amount of the compound of formula (I) or formula (II) and the amount of the suitable pharmaceutical agent that when taken together or sequentially have a combined effect that is therapeutically effective. Further, it will be recognized by one skilled in the art that in the case of co-therapy with a therapeutically effective amount, as in the example above, the amount of the compound of formula (I) or formula (II) and/or the amount of the suitable pharmaceutical agent individually may or may not be therapeutically effective.

As used herein, the terms "**co-therapy**" and "**combination therapy**" shall mean treatment of a subject in need thereof by administering one or more compounds of formula (I) or formula (II) in combination with one or more suitable pharmaceutical agent(s), wherein the compound(s) of formula (I) or formula (II) and the suitable pharmaceutical agent(s) are administered by any suitable means, simultaneously, sequentially, separately or in a single pharmaceutical formulation. Where the compound(s) of formula (I) or formula (II) and the suitable pharmaceutical agent(s) are administered in separate dosage forms, the number of dosages administered per day for each compound may be the same or different. The compound(s) of formula (I) or formula (II) and the suitable pharmaceutical agent(s) may be administered via the same or different routes of administration. Examples of suitable methods of administration include, but are not limited to, oral, intravenous (iv), intramuscular (im), subcutaneous (sc), transdermal, and rectal. Compounds may also be administered directly to the nervous system including, but not limited to, intracerebral, intraventricular, intracerebroventricular, intrathecal, intracisternal, intraspinal and / or peri-spinal routes of administration by delivery via intracranial or intravertebral needles and / or catheters with or without pump devices. The compound(s) of formula (I) or formula (II) and the suitable pharmaceutical agent(s) may be administered according to simultaneous or alternating regimens, at the same or different times during the course of the therapy, concurrently in divided or single forms.

As used herein, unless otherwise noted, the term "**suitable pharmaceutical agent**" is intended to mean any pharmaceutical agent that has one or more of the following properties: antioxidant activity; NMDA receptor antagonist activity, augmentation of endogenous GABA inhibition; NO synthase inhibitor activity; iron binding ability, e.g., an iron chelator; calcium binding ability, e.g., a Ca (II) chelator; zinc binding ability, e.g., a Zn (II) chelator; the ability to effectively block sodium or calcium ion channels, or to open potassium or chloride ion channels in the CNS of a patient, including known AEDs or are therapeutic agents useful in the treatment of Substance Abuse and addiction, including, methadone, disulfiram, bupropion, antipsychotics, antidepressants, benzodiazepines, buspirone, naloxone or naltrexone.
Preferably, the suitable pharmaceutical agent antagonizes NMDA receptors by binding to the NMDA receptors (e.g., by binding to the glycine binding site of the NMDA receptors) and/or the agent augments GABA inhibition by decreasing glial GABA uptake.

In addition, the "**suitable pharmaceutical agent**" may be any agent known to suppress seizure activity even if that compound is not known to inhibit epileptogenesis. Such agents would include any effective AED or anti-convulsant known to one of skill in the art or discovered in the future, for example suitable agents include carbamazepine, clobazam, clonazepam, ethosuximide, felbamate, gabapentin, lamotigine, levetiracetam, oxcarbazepine, phenobarbital, phenytoin, pregabalin; primidone, retigabine, talampanel, tiagabine, topiramate, valproate, vigabatrin, zonisamide, benzodiazepines, barbiturates or sedative hypnotics.

The term "**epilepsy**" refers to a disorder of brain function characterized by the periodic and unpredictable occurrence of seizures (See, The Treatment of Epilepsy, Principles & Practice, Third Edition, Elaine Wyllie, M.D. Editor, Lippincott Williams & Wilkins, 2001; Goodman & Gilman's The Pharmacological Basis of Therapeutics, 9th edition, 1996) Seizures that occur without evident provocation are classified as epileptic. Epilepsy may be idiopathic or may be related to some kind of injury, malformation or damage to the central nervous at any stage of life. A subject is typically considered to suffer from epilepsy upon experiencing two or more seizures that occur more than 24 hours apart.
Clinically, an epileptic seizure results from a sudden and abnormal electrical discharge originating from a collection of interconnected neurons in the brain or elsewhere in the nervous system. Depending on the type of epilepsy involved, the resulting nerve cell activity may be manifested by a wide variety of clinical symptoms such as uncontrollable motor movements, changes in the patient's level of consciousness.Epilepsy and epileptic seizures and syndromes may be classified in a variety of ways (See, The Treatment of Epilepsy, Principles & Practice, Third Edition, Elaine Wyllie, M.D. Editor, Lippincott Williams & Wilkins, 2001). However, as used herein the terms; **"epilepsy", "epileptic seizures"** and **"epileptic syndromes"** are meant to include all known types of epileptic seizures and syndromes including; partial seizures, including simple, complex and partial seizures evolving to generalized tonic-clonic convulsions and generalized seizures, both convulsive and nonconvulsive and unclassified epileptic seizures.

**"Epileptogenesis"** of the **"epileptogenic process"** generally consists of two phases. It is intended that the methods of the present invention include prophylactic and / or therapeutic administration of any of the compounds described herein at either the first or second epileptogenic stage or preceding these stages to treat, inhibit, prevent, arrest or reverse the subsequent development of epilepsy or other analogous seizure-related disorder in a subject in need thereof.

The first epileptogenic stage is known as the initial insult or injury stage. The initial insult or injury is commonly a brain-damaging injury caused by one or more of a multitude of possible factors including, for example, traumatic brain injury, including blunt and penetrating head trauma or a neurosurgical procedure; CNS infection, such as, for example, bacterial meningitis, viral encephalitis, bacterial cerebral abscess or neurocysticercosis); cerebrovascular disease (such as stroke or brain tumor including, for example, malignant gliomas; neurosurgery (such as for example craniotomies) and status epilepticus. In some instances, the initial insult will be a result of developmental problems before birth (such as, but not limited to, birth asphyxia, intracranial trauma during birth, metabolic disturbances or congenital malformations of the brain) or as the result of genetic determinants.
The second epileptogenic stage is known as the "latency stage". The second epileptogenic stage includes the process of neuronal restructuring, which is characterized by recurrent seizures (e.g. symptomatic epilepsy) or by symptoms shown in analogous seizure-related disorders. The epileptogenic process can also be observed among persons actually suffering from epilepsy or analogous seizure-related disorders. The seizures experienced by persons suffering from epilepsy are themselves epileptogenic in that they tend to make the occurrence of subsequent seizures more likely or extend the area of nervous tissue that is subject to seizure activity or make the seizure disorder more resistant to treatment. The consequences of this process, for a patient who has a seizure disorder, is that the seizures tend to become more frequent and more severe and often more resistant to treatment with conventional AED's. In a similar manner, the related seizure-like response in neurological or psychiatric disorders analogous to epilepsy may become increasingly severe over time or resistant to treatment as the disorder matures.

Phase 1 epileptogenesis can be initiated by factors other than those listed above, such as by the ingestion of compounds with epileptogenic potential, e.g., psychotropic medications such as, for example, tricyclic antidepressants, clozapine, and lithium and the like. The methods of the present invention are also intended to treat, prevent, arrest, inhibit and / or reverse the development of epileptogenesis which has been initiated by factors which tend to increase the potential for a subject to become epileptogenic.

The compounds of formula (I) and formula (II) as described herein are useful in the treatment of epilepsy and analogous seizure related disorders. In addition, the compounds of formula (I) and formula (II) described herein are useful to suppress, control and prevent the process of epileptogenesis that results in the worsening, clinical progression or increasing resistance to treatment of epilepsy and related seizure disorders or to the de-novo initiation of these disorders and their symptoms as a result of some form of injury or trauma to the nervous system.
Thus, in an embodiment, the present invention is directed to methods which allow the clinician to treat the symptoms of epilepsy, other seizure disorders and/or symptoms of analogous seizure related disorders while simultaneously inhibiting the epileptogenic process that is responsible for the worsening, progression, extension or increasing treatment resistance of the underlying disease process. The method comprises, the prophylactic or therapeutic administration, to a subject in need thereof, of an anti-epileptogenesis effective amount or dose of a compound of formula (I) and / or formula (II) as described herein to the subject that simultaneously treats and prevents the seizures or other symptoms of the disorder and, in addition, is able to arrest, inhibit and reverse the process of epileptogenesis in the subject.

In certain embodiments, the subject or patient in need of treatment (preferably the subject or patient in need of treatment with an AEGD) may be a subject who has already shown the symptoms of epilepsy, i.e., seizures or convulsions or a subject or patient which has shown the symptoms of an analogous seizure-related disorder (e.g. mood cycling, impulsive behavior, addictive behavior and the like) before or at the time of administration. Therefore, in one aspect, the present invention provides an improved method for treating and preventing seizures and the symptoms of seizure-related disorders in a subject in need thereof. The method includes the step of prophylactically or therapeutically administering to the subject in need thereof a therapeutically effective amount of a compound of formula (I) and / or formula (II) as described herein that treats and prevents the occurrence of seizures, convulsions or seizure-related disorders.
In certain other embodiments, the subject or patient in need of treatment (preferably the subject or patient in need of treatment with an AEGD) may be a subject who has not shown the symptoms of epilepsy, i.e., seizures or convulsions or the symptoms of an analogous seizure-related disorder prior to the time of administration. In this embodiment, the subject or patient will be determined to be at risk for developing epilepsy or an analogous seizure-related disorder at the time of administration and on this basis will be considered to be a patient in need of treatment with an AEGD. In this aspect, the invention provides a method for arresting, inhibiting and / or reversing epileptogenesis. The method includes the step of prophylactically or therapeutically administering to the subject in need thereof a prophylactically or therapeutically effective amount of any of the compounds described herein to the subject that treats, prevents, arrests, inhibits and reverses epileptogenesis.

By suppressing the process of epileptogenesis the development of a seizure disorder or a related disorder can be prevented in a subject who has sustained some form of injury or damage to the nervous system or who is otherwise at risk. Accordingly, the present invention provides methods for treating, preventing, arresting, inhibiting and / or reversing epileptogenesis in a subject in need thereof comprising administering to the subject a prophylactically or therapeutically effective amount of a composition that comprises at least one compound of formula (I) and / or formula (II).

In an embodiment of the present invention the method are advantageously used to treat a patient who is not suffering or known to be suffering from a condition that is known in the art to be effectively treated with presently known anticonvulsant or antiepileptic (AED) medications. These conditions include but are not limited to analogous seizure-related disorder(s). In these cases the decision to use the methods and compounds of the present invention would be made on the basis of determining if the patient is a "patient in need of treatment with an anti-epileptogenic drug (AEGD)" as that term is defined above.

The methods of the present invention are directed toward treating epileptogenesis in a subject who is at risk of developing epilepsy or a seizure related disorder or analogous seizure related disorder (s) but does not have epilepsy or clinical evidence of seizures. A subject who is at risk of developing epilepsy or an analogous seizure related disorder (s) but who does not have epilepsy or other seizure disorder or an analogous seizure related disorder (s) can be a subject who has not yet been diagnosed with epilepsy or an analogous seizure related disorder (s) but who is at greater risk than the general population for developing epilepsy or an analogous seizure related disorder (s). This "greater risk" may be determined by the recognition of any factor in a subject's, or their family's medical history, physical exam or testing that is indicative of a greater than average risk for developing epilepsy or an analogous seizure related disorder (s). Therefore this determination that a patient may be at a "greater risk" by any available means can be used to determine whether the patient should be treated with the methods of the present invention.
Patients who are at greater risk include those who have not suffered damage or injury to their central nervous system but have a high likelihood of such damage or injury either because of their medical condition or their environment. This would include patients with a history of Transient ischemic Attacks (TIA's) or known carotid artery stenosis or simply known significant arteriosclerosis as well as patients about to undergo a neurosurgical procedure. In addition, individuals likely to suffer neurological damage due to war or sports injury could be prophylactically administered compounds of the invention; this would include soldiers in combat or athletes in violent contact sports such as boxing.
The subjects who may benefit from treatment by the methods of the present invention can be identified using accepted screening methods to determine risk factors associated with epileptogenesis, epilepsy or other seizure disorders or an analogous seizure related disorder. A determination that a subject has, or may be at risk for developing, epilepsy, another seizure disorder or an analogous seizure related disorder would also include, for example, a medical evaluation that includes a thorough history, a physical examination, and a series of relevant bloods tests. It can also include an electroencephalogram (EEG), computer tomography (CT), magnetic resonance imaging (MRI) or positron emission tomography (PET). A determination of an increased risk of developing epilepsy or an analogous seizure related disorder may also be made by means of genetic testing, including gene expression profiling or proteomics techniques. (See, Schmidt, D. Rogawski, M. A. Epilepsy Research 50; 71-78 (2002), and Loscher, W, Schmidt D. Epilepsy Research 50; 3-16 (2002))
These screening methods include, for example, conventional medical work-ups to determine risk factors that may be associated with epileptogenesis including but not limited to, for example, head trauma, either closed or penetrating, neurosurgical procedures, CNS infections, bacterial or viral, Trigeminal Neuralgia, cerebrovascular disease, including stroke or a history of TIA's, brain tumors, brain edema, cysticercosis, porphyria, metabolic encephalopathy, drug withdrawal including but not limited to sedative-hypnotic or alcohol withdrawal, abnormal perinatal history including anoxia at birth or birth injury of any kind, cerebral palsy, learning disabilities, hyperactivity, history of febrile convulsions, history of status epilepticus, family history of epilepsy or any a seizure related disorder, inflammatory disease of the brain or blood vessels including lupus, drug intoxication, either direct or by placental transfer, including cocaine and methamphetamine toxicity, parental consanguinity, and treatment with medications that lower seizure threshold including psychotropic medications such as antidepressant or anti psychotic medications.

The determination of which patients may benefit from treatment with an AEGD in patients who have no clinical signs or symptoms of epilepsy or other seizure disorder or an analogous seizure related disorder may be based on a variety of **"surrogate markers"** or **"biomarkers".** Such biomarkers include, but are not limited to, gene or protein expression profiles in tissue, blood or CSF or the presence of genetic markers such as SNP's.
As used herein, the terms **"surrogate marker"** and **"biomarker"** are used interchangeably and refer to any anatomical, biochemical, structural, electrical, genetic or chemical indicator or marker that can be reliably correlated with the present existence or future development of epilepsy or a seizure disorder or an analogous seizure related disorder. In some instances, brain-imaging techniques, such as computer tomography (CT), magnetic resonance imaging (MRI) or positron emission tomography (PET), or other neurological imaging techniques can be used to determine whether a subject is at risk for developing one of the above disorders.
Examples of suitable biomarkers for the methods of this invention include, but are not limited to: the determination by MRI, CT or other imaging techniques, of sclerosis, atrophy or volume loss in the hippocampus or the presence of mesial temporal sclerosis (MTS) or similar relevant anatomical pathology; the detection in the patient's blood, serum or tissues of a molecular species such as a protein or other biochemical biomarker, e.g., elevated levels of ciliary neurotrophic factor (CNTF) or elevated serum levels of a neuronal degradation product; or other evidence from surrogate markers or biomarkers that the patient is in need of treatment with an anti-epileptogenic drug, e.g. an EEG suggestive of a seizure disorder or an analogous seizure related disorder (s) epilepsy related seizure like neurological phenomenon or seizure rotated disorder.
It is expected that many more such biomarkers utilizing a wide variety of detection techniques will be developed in the future. It is intended that any such marker or indicator of the existence or possible future development of a seizure disorder, epilepsy or an analogous seizure related disorder, as the latter term is used herein, may be used in the methods of this invention for determining the need for treatment with the compositions and methods of this invention.

In an embodiment of the present invention, treatment is directed at patients who had epilepsy or an epilepsy related seizure like neurological phenomenon or an analogous seizure related disorder, as defined above, and by taking advantage of the ability of the compounds of the present invention to reverse epileptogenesis would allow the gradual reduction in the dosages of maintenance medication or intensity of treatment required to control the clinical manifestations of the patient's epilepsy or epilepsy related seizure like neurological phenomenon or analogous seizure related disorder, as defined above.

Therefore, as the treatment with the methods of the present invention produced improvement in the underlying disorder, the patient could be withdrawn from their maintenance medication including the compounds of the present invention themselves if they are being used as sole therapy. Thus, a patient with epilepsy on a maintenance therapy of a conventional AED could be withdrawn from the AED after the treatment with one or more of the compounds of the present invention had reversed the underlying epileptic disorder.
One of skill in the art could determine how rapidity to conduct the taper based on clinical signs and symptoms including EEG's, breakthrough seizures or other appropriate biomarkers of the underlying disorder.

The present invention relates to methods for the treatment, prevention, suppression, arrest and / or inhibition of epileptogenesis comprising adminestering to a subject in need thereof, preferable a subject in need of treatment with an AEGD, a therapeutically and / or prophylactically effective amount of a compound of formula (I) and / or formula (II), as described herein.

In an embodiment of the present invention R¹ is selected from the group consisting of hydrogen and methyl. In another embodiment of the present invention R² is selected from the group consisting of hydrogen and methyl. In yet another embodiment of the present invention R¹ and R² are each hydrogen or R¹ and R² are each methyl.

In an embodiment of the present invention -(CH₂)ₐ- is selected from the group consisting of -CH₂- and -CH₂-CH₂-. In another embodiment of the present invention -(CH₂)ₐ- is -CH₂-.

In an embodiment of the present R⁴ is selected from the group consisting of hydrogen and methyl, preferably, R⁴ is hydrogen.

In an embodiment of the present invention a is 1.

In an embodiment of the present invention b is an integer from 0 to 2. In another embodiment of the present invention c is an integer from O to 2. In another embodiment of the present invention b is an integer from 0 to 1. In another embodiment of the present invention c is an integer from O to 1. In yet another embodiment of the present invention the sum of b and c is an integer form 0 to 2, preferably an integer form 0 to 1. In yet another embodiment of the present invention b is an integer from 0 to 2 and c is 0.

In an embodiment of the present invention, 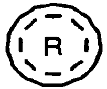 is selected from the group consisting of 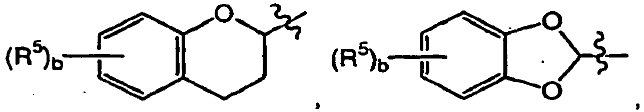 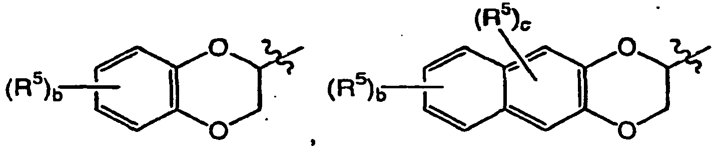 and 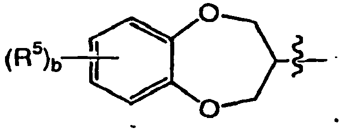 In another embodiment of the present invention, 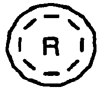 is selected from the group consisting of 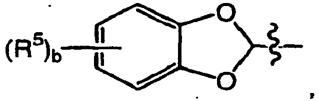 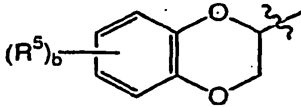 and

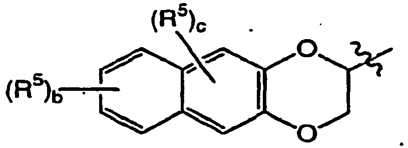

In an embodiment of the present invention, 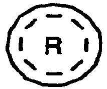 is selected from the group consisting of 2-(2,3-dihydro-benzo[1,4]dioxinyl), 2-(benzo[1,3]dioxolyl), 3-(3,4-dihydro-benzo[1,4]dioxepinyl), 2-(6-chloro-2,3-dihydro-benzo[1,4]dioxinyl), 2-(6-fluoro-2,3-dihydro-benzo[1,4]dioxinyl), 2-(chromanyl), 2-(5-fluoro-2,3-dihydro-benzo[1,4]dioxinyl), 2-(7-chloro-2,3-dihydro-benzo[1,4]dioxinyl), 2-(6-chloro-benzo[1,3]dioxolyl), 2-(7-nitro-2,3-dihydro-benzo[1,4]dioxinyl), 2-(7-methyl-2,3-dihydro-benzo[1,4]dioxinyl), 2-(5-chloro-2,3-dihydrobenzo[1,4]dioxinyl), 2-(6-bromo-2,3-dihydro-benzo[1,4]dioxinyl), 2-(6,7-dichloro-2,3-dihydro-benzo[1,4]dioxinyl), 2-(8-chloro-2,3-dihydro-benzo[1,4]dioxinyl), 2-(2,3-dihydro-naphtho[2,3-b][1,4]dioxinyl) and 2-(4-methyl-benzo[1,3]dioxolyl).

In another embodiment of the present invention, 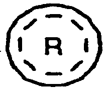 is selected from the group consisting 2-(benzo[1,3]dioxolyl), 2-(2,3-dihydrobenzo[1,4]dioxinyl), 2-(6-chloro-2,3-dihydro-benzo[1,4]dioxinyl), 2-(7-chloro-2,3-dihydro-benzo[1,4]dioxinyl), 2-(7-methyl-2,3-dihydro-benzo[1,4]dioxinyl), 2-(6-bromo-2,3-dihydro-benzo[1,4]dioxinyl) and 2-(6,7-dichloro-2,3-dihydro-benzo[1,4]dioxinyl). In another embodiment of the present invention, 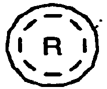 is selected from the group consisting of 2-(2,3-dihydro-benzo[1,4]dioxinyl), 2-(7-methyl-2,3-dihydro-benzo[1,4]dioxinyl) and 2-(6-bromo-2,3-dihydrobenzo[1,4]dioxinyl).

In an embodiment of the present invention R⁵ is selected from the group consisting of halogen and lower alkyl. In another embodiment of the present invention R⁵ is selected from chloro, fluoro, bromo and methyl.

In an embodiment of the present invention, the stereo-center on the compound of formula (I) is in the S-configuration. In another embodiment of the present invention, the stereo-center on the compound of formula (I) is in the R-configuration.
In an embodiment of the present invention the compound of formula (I) is present as an enantiomerically enriched mixture, wherein the % enantiomeric enrichment (% ee) is greater than about 75%, preferably greater than about 90%, more preferably greater than about 95%, most preferably greater than about 98%.

Additional embodiments of the present invention, include those wherein the substituents selected for one or more of the variables defined herein (i.e. R¹, R², R³, R⁴, X-Y and A) are independently selected to be any individual substituent or any subset of substituents selected from the complete list as defined herein.

Representative compounds of the present invention, are as listed in Tables 1 below. Additional compounds of the present invention are as listed in Table 3. In Tables 1 and 2 below, the column headed "stereo" defines the stereo-configuration at the carbon atom of the heterocycle attached at the starred bond. Where no designation is listed, the compound was prepared as a mixture of stereo-configurations. Whens an "R" or "S" designation is listed, the stereo-configuration was based on the enantiomerically enriched starting material.

**Table 1: Representative Compounds of Formula (I)**

| | | | | | | |
|---|---|---|---|---|---|---|
| | 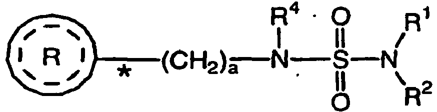 | | | | | |

| **ID No.** | 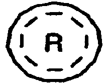 | **Stereo** | (**CH₂)ₐ** | **NR⁴** | **R¹** | **R²** |
|---|---|---|---|---|---|---|
| 1 | 2-(2,3-dihydrobenzo[1,4]dioxinyl) | | CH₂ | NH | H | H |
| 2 | 2-(benzo[1,3]dioxolyl) | | CH₂ | NH | H | H |
| 3 | 3-(3,4-dihydro-2H-benzo[1,4]dioxepinyl) | | CH₂ | NH | H | H |
| 4 | 2-(2,3-dihydrobenzo[1,4]dioxiriyl) | S | CH₂ | NH | H | H |
| 5 | 2-(2,3-dihydrobenzo[1,4]dioxinyl) | R | CH₂ | NH | H | H |
| 6 | 2-(2,3-dihydrobenzo[1,4]dioxinyl) | | CH₂ | NH | methyl | methyl |
| 7 | 2-(2,3-dihydrobenzo[1,4]dioxinyl) | | CH₂ | N(CH₃) | H | H |
| 8 | 2-(6-chloro-2,3-dihydrobenzo[1,4]dioxinyl) | S | CH₂ | NH | H | H |
| 9 | 2-(6-fluoro-2,3-dihydrobenzo[1,4]dioxinyl) | S | CH₂ | NH | H | H |
| 10 | 2-(chromanyl) | | CH₂ | NH | H | H |
| 13 | 2-(5-fluoro-2,3-dihydrobenzo[1,4]dioxinyl) | S | CH₂ | NH | H | H |
| 14 | 2-(7-chloro-2,3-dihydrobenzo[1,4]dioxinyl | S | CH₂ | NH | H | H |
| 15 | 2-(6-chlorobenzo[1,3]dioxolyl) | | CH₂ | NH | H. | H |
| 16 | 2-(2,3-dihydrobenzo[1,4]dioxinyl) | | CH₂CH₂ | NH | H | H |
| 18 | 2-(7-nitro-2,3-dihydrobenzo[1,4]dioxinyl) | S | CH₂ | NH | H | H |
| 19 | 2-(7-methyl-2,3-dihydrobenzo[1,4]dioxinyl) | S | CH₂ | NH | H | H |
| 20 | 2-(5-chloro-2,3-dihydrobenzo[1,4]dioxinyl) | S | CH₂ | NH | H | H |
| 22 | 2-(8-methoxy-2,3-dihydrobenzo[1,4]dioxinyl) | S | CH₂ | NH | H | H |
| 24 | 2-(6-bromo-2,3-dihydrobenzo[1,4]dioxinyl) | S | CH₂ | NH | H | H |
| 29 | 2-(6,7-dichloro-2,3-dihydrobenzo[1,4]dioxinyl) | S | CH₂ | NH | H | H |
| 30 | 2-(8-chloro-2,3-dihydrobenzo[1,4]dioxinyl) | S | CH₂ | NH | H | H |
| 33 | 2-(2,3-dihydronaphtho[2,3-b][1,4]dioxinyl) | S | CH₂ | NH | H | H |
| 35 | 2-(4-methylbenzo[1,3]dioxolyl) | | CH₂ | NH | H | H |

**Table 2: Additional Compounds of the Present Invention**

| | | | | | | |
|---|---|---|---|---|---|---|
| 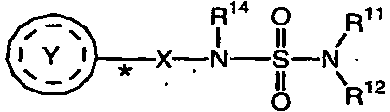 | | | | | | |

| **ID No.** | 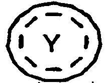 | **Stereo** | **X** | **NR¹⁴** | **R¹¹** | **R¹²** |
|---|---|---|---|---|---|---|
| 23 | 2-(5-methoxy-2,3-dihydrobenzo[1,4]dioxinyl) | S | CH₂ | NH | H | H |
| 26 | 2-(6-methylcarbonyl-2,3-dihydrobenzo[1,4]dioxinyl) | S | CH₂ | NH | H | H |
| 32 | 2-(6-methoxycarbonyl-2,3-dihydrobenzo[1,4]dioxinyl) | S | CH₂ | NH | H | H |
| 34 | 2-(6-hydroxymethyl-2,3-dihydrobenzo[1,4]dioxinyl) | S | CH₂ | NH | H | H |
| 36 | 2-(7-amino-2,3-dihydrobenzo[1,4]dioxinyl) | S | CH₂ | NH | H | H |

As used herein, unless otherwise noted, **"halogen"** shall mean chlorine, bromine, fluorine and iodine.

As used herein, unless otherwise noted, the term **"alkyl"** whether used alone or as part of a substituent group, includes straight and branched chains. For example, alkyl radicals include methyl, ethyl, propyl, isopropyl, butyl, isobutyl, sec-butyl, t-butyl and pentyl. Unless otherwise noted, "lower" when used with alkyl means a carbon chain composition of 1-4 carbon atoms.

As used herein, unless otherwise noted, **"alkoxy"** shall denote an oxygen ether radical of the above described straight or branched chain alkyl groups. For example, methoxy, ethoxy, n-propoxy, t-butoxy and n-hexyloxy.

As used herein, the notation "*" shall denote the presence of a stereogenic center.

When a particular group is **"substituted"** (e.g., alkyl, aryl, etc.), that group may have one or more substituents, preferably from one to five substituents, more preferably from one to three substituents, most preferably from one to two substituents, independently selected from the list of substituents.

With reference to substituents, the term **"independently"** means that when more than one of such substituents is possible, such substituents may be the same or different from each other.

Under standard nomenclature used throughout this disclosure, the terminal portion of the designated side chain is described first, followed by the adjacent functionality toward the point of attachment. Thus, for example, a **"phenyl-alkylamino-carbonyl-alkyl"** substituent refers to a group of the formula

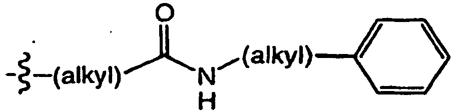

Abbreviations used in the specification, particularly the Schemes and Examples, are as follows:

| | |
|---|---|
| DCC | = Dicyclohexyl Carbodiimide |
| DCE | = Dichloroethane |
| DCM | = Dichloromethane |
| DIPEA or DIEA | = Diisopropylethylamine |
| DMF | = N,N-Dimethylformamide |
| DMSO | = Dimethylsulfoxide |
| EDC | = Ethylcarbodiimide |
| Et₃N or TEA | = Triethylamine |
| Et₂O | = Diethyl ether |
| EA or EtOAc | = Ethyl acetate |
| EtOH | = Ethanol |
| IPA | = 2-propanol |
| Hept | = Heptane |
| HOBT | = 1-Hydroxybenzotriazole |
| HPLC | = High Pressure Liquid Chromatography |
| LAH | = Lithium Aluminum Hydride |
| M or MeOH | = Methanol |
| NMR | = Nuclear Magnetic Resonance |
| Pd-C | = Palladium on Carbon Catalyst |
| RP HPLC | = Reverse Phase High Pressure Liquid Chromatography |
| RT or rt | = Room temperature |
| TEA | = Triethylamine |
| TFA | = Trifluoroacetic Acid |
| THF | = Tetrahydrofuran |
| TLC | = Thin Layer Chromatography |

Where the compounds according to this invention have at least one chiral center, they may accordingly exist as enantiomers. Where the compounds possess two or more chiral centers, they may additionally exist as diastereomers. It is to be understood that all such isomers and mixtures thereof are encompassed within the scope of the present invention. Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

For use in medicine, the salts of the compounds of this invention refer to non-toxic **"pharmaceutically acceptable salts."** Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts. Thus, representative pharmaceutically acceptable salts include the following:
acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

Representative acids and bases which may be used in the preparation of pharmaceutical acceptable salts include the following:
acids including acetic acid, 2,2-dichloroactic acid, acylated amino acids, adipic acid, alginic acid, ascorbic acid, L-aspartic acid, benzenesulfonic acid, benzoic acid, 4-acetamidobenzoic acid, (+)-camphoric acid, camphorsulfonic acid, (+)-(1 S)-camphor-10-sulfonic acid, capric acid, caproic acid, caprylic acid, cinnamic acid, citric acid, cyclamic acid, dodecylsulfuric acid, ethane-1,2-disulfonic acid, ethanesulfonic acid, 2-hydrocy-ethanesulfonic acid, formic acid, fumaric acid, galactaric acid, gentisic acid, glucoheptonic acid, D-gluconic acid, D-glucoronic acid, L-glutamic acid, α-oxo-glutaric acid, glycolic acid, hipuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5-disulfonic acid, 1-hydroxy-2-naphthoic acid, nicotine acid, nitric acid, oleic acid, orotic acid, oxalic acid, palmitric acid, pamoic acid, phosphoric acid, L-pyroglutamic acid, salicylic acid, 4-amino-salicylic acid, sebaic acid, stearic acid, succinic acid, sulfuric acid, tannic acid, (+)-L-tartaric acid, thiocyanic acid, p-toluenesulfonic acid and undecylenic acid; and
bases including ammonia, L-arginine, benethamine, benzathine, calcium hydroxide, choline, deanol, diethanolamine, diethylamine, 2-(diethylamino)-ethanol, ethanolamine, ethylenediamine, N-methyl-glucamine, hydrabamine, 1 H-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

Compounds of formula (I) may be prepared according to the process outlined in Scheme 1.

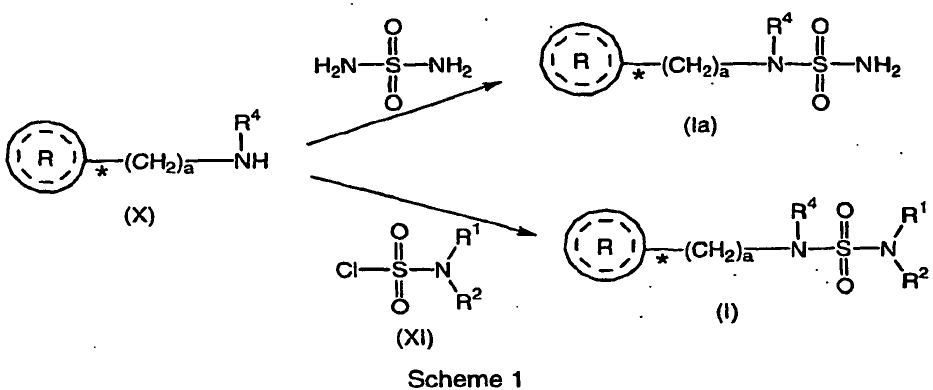

Accordingly, a suitably substituted compound of formula (X), a known compound or compound prepared by known methods, is reacted with sulfamide, a known compound, preferably wherein the sulfamide is present in an amount in the range of about 2 to about 5 equivalents, in an organic solvent such as THF, dioxane, and the like, preferably at an elevated temperature in the range of about 50°C to about 100°C, more preferably at about reflux temperature, to yield the corresponding compound of formula (Ia).

Alternatively, a suitably substituted compound of formula (X), a known compound or compound prepared by known methods, is reacted with a suitably substituted compound of formula (XI), a known compound or compound prepared by known methods, in the presence of a base such as TEA, DIPEA, pyridine, and the like, in an organic solvent such as DMF, DMSO, and the like, to yield the corresponding compound of formula (I).

Compounds of formula (X) wherein 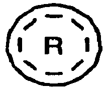 is 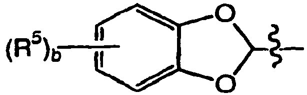 may be prepared according to the process outlined in Scheme 2.

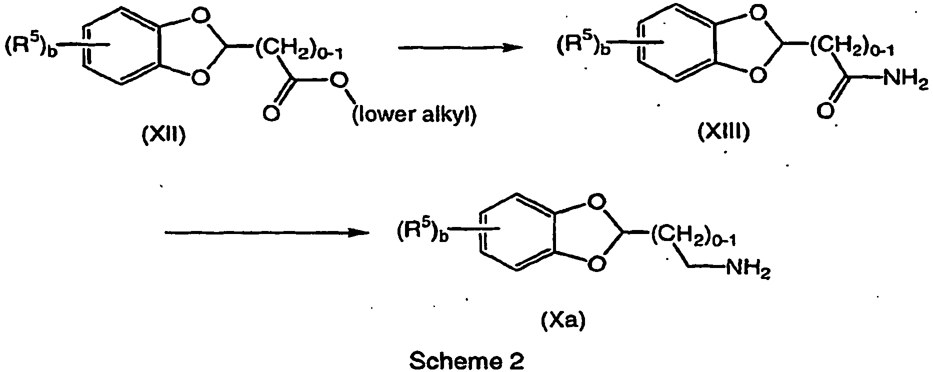

Accordingly, a suitably substituted compound of formula (XII), a known compound or compound prepared by known method (for example as described in Scheme 3 above) is reacted with NH₄OH, a known compound, optionally in an organic solvent such as acetonitrile, and the like, to yield the corresponding compound of formula (XIII).

The compound of formula (XIII) is reacted with a suitably selected reducing agent, such as LAH, and the like, and the like, in an organic solvent such as THF, diethyl ether, and the like, to yield the corresponding compound of formula (Xa).

Compounds of formula (X) wherein 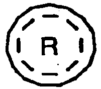 is selected from 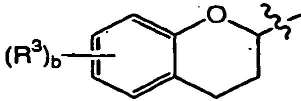 may be prepared according to the process outlined in Scheme 3.

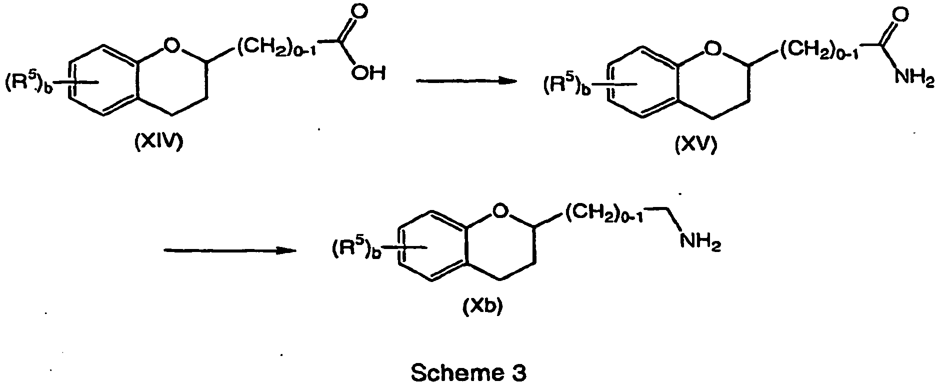

Accordingly, a suitably substituted compound of formula (XIV), a known compound or compound prepared by known methods, is reacted with NH₄OH, in the presence of a coupling agent such as DCC, and the like, optionally in an organic solvent such as acetonitrile, and the like, to yield the corresponding compound of formula (XV).
The compound of formula (XV) is reacted with a suitably selected reducing agent, such as LAH, and the like, in an organic solvent such as THF, diethyl ether, and the like, to yield the corresponding compound of formula (Xb).

Compounds of formula (X) wherein 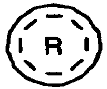 is selected from 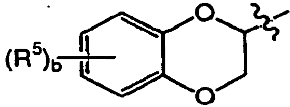 and wherein a is 2, may be prepared according to the process outlined in Scheme 4.

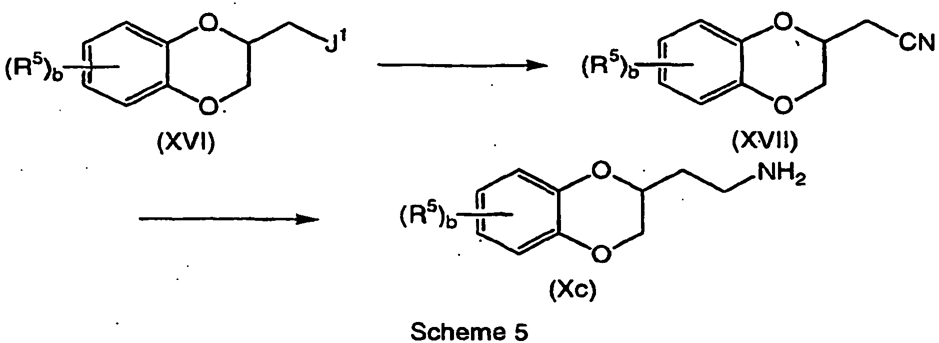

Accordingly, a suitably substituted compound of formula (XVI) wherein J¹ is a suitable leaving group such as Br, Cl, I, tosyl, mesyl, triflyl, and the like, a known compound or compound prepared by known methods (for example, by activating the corresponding compound wherein J¹ is OH), is reacted with a cyanide such as potassium cyanide, sodium cyanide, and the like, in an organic solvent such as DMSO, DMF, THF, and the like, to yield the corresponding compound of formula (XVII).
The compound of formula (XVII) is reduced according to known methods, for example by reacting with a suitable reducing agent such as LAH, borane, and the like, to yield the corresponding compound of formula (Xc).

Compounds of formula (X) wherein 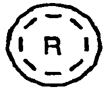 is selected from 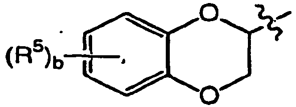 and wherein a is 1, may be prepared according to the process outlined in Scheme 5.

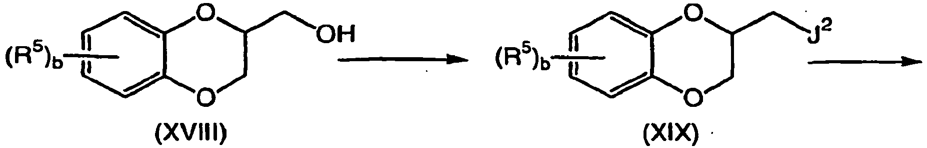

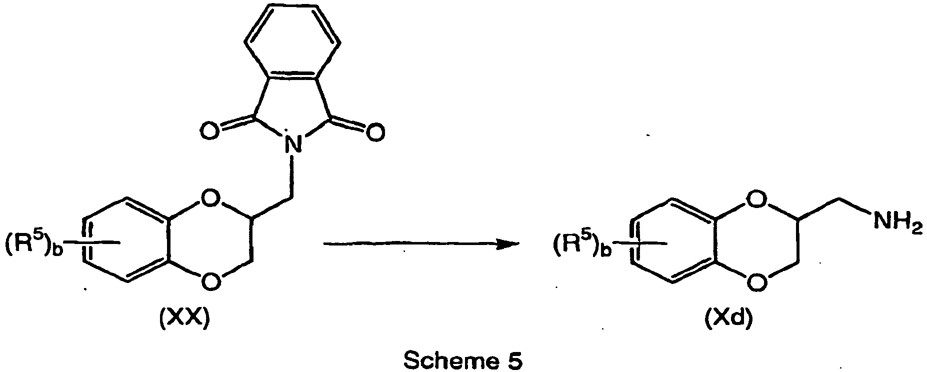

Accordingly, a suitably substituted compound of formula (XVIII), a known compound or compound prepared by known methods is activated, according to known method, to yield the corresponding compound of formula (XIX), wherein J² is a suitable leaving group, such tosylate, Cl, Br, I, mesylate, triflate, and the like.

The compound of formula (XIX) is reacted with a phthalimide salt such as potassium phthlimide, sodium phthalimide, and the like, in an organic solvent such as DMF, DMSO, acetonitrile, and the like, preferably, at an elevated temperature in the range of from 50°C to about 200°C, more preferably, at about reflux temperature, to yield the corresponding compound of formula (XX).

The compound of formula (XX) is reacted with N₂H₄, a known compound, in an organic solvent-such as ethanol, methanol, and the like, preferably, at an elevated temperature in the range of from about 50°C to about 100°C, more preferably, at about reflux temperature, and the like, to yield the corresponding compound of formula (Xd).

One skilled in the art will recognize that compounds of formula (X) wherein 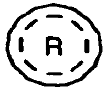 is selected from 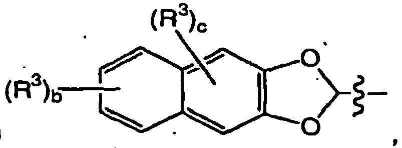 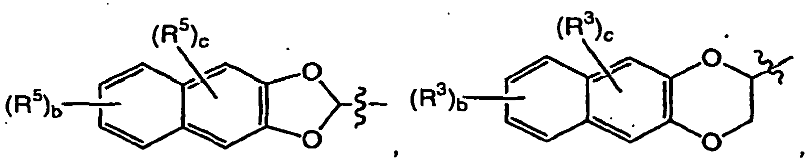 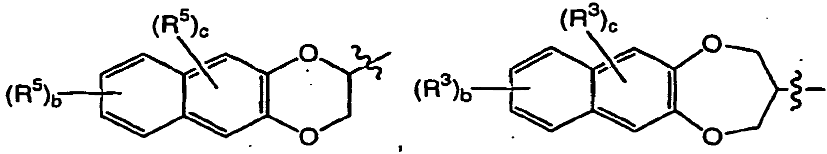 or 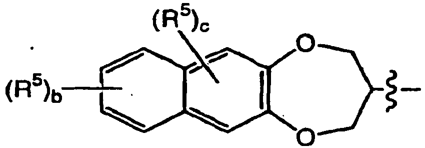 may be similarly prepared according to known methods or for example, according to the processes outlined in Schemes 2 through 5 above, by selecting and substituting the corresponding naphthyl-fused compounds for the benzo-fused starting materials.

One skilled in the art will further recognize that wherein a single enantiomer (or a mixture of enantiomers wherein one enantiomer is enriched) of a compound of formula (X) is desired, the above processes as described in Schemes 1 through 5 may be applied by substituting the corresponding single enantiomer (or mixture of enantiomers wherein one enantiomer is enriched) for the appropriate starting material.

One skilled in the art will recognize that wherein a reaction step of the present invention may be carried out in a variety of solvents or solvent systems, said reaction step may also be carried out in a mixture of the suitable solvents or solvent systems.

Where the processes for the preparation of the compounds according to the invention give rise to mixture of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as (-)-di-p-toluoyl-D-tartaric acid and/or (+)-di-p-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral HPLC column.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

The present invention provides methods of treating epileptogenesis, regardless of underlying cause and stage of development, comprising administering to a subject in need thereof a therapeutically effective amount of a compound of formula (I) or formula (II) as described herein. The methods of this invention therefore provide the ability to suppress seizures, convulsions or the symptoms of an analogous seizure related disorder while simultaneously preventing the process of epileptogenesis so as to prevent the progression or worsening of the underlying disease or the recruitment by the process of epileptogenesis of non seizure prone nervous tissue. In order to accomplish this objective the compounds or compositions of this invention must be used in the correct therapeutically effective amount or dose, as described below

Optimal dosages and schedules to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

In an embodiment of the present invention, the treatment regimen with a compound of formula (I) and / or formula (II) can commence in a subject or patient who has had seizures sufficient to justify a diagnoses of epilepsy. In this embodiment the compounds of the invention may be simultaneously employed as AED's to suppress seizures in a patient with a recognized seizure disorder or epilepsy. However, in this context; according to the methods of the invention, these compounds are used in the proper dosage ranges in order to, in addition, provide an anti-epileptogenesis effect (AEGD effect) and prevent the extension or expansion of the nervous tissue subject to seizure activity and the consequent worsening of the disease.

In another embodiment, the treatment regimen with the compounds of the present invention can commence, for example, after a subject suffers from a brain damaging injury or other initial insult but before the subject is diagnosed with epilepsy, e.g., before the subject has a first or second seizure. In one embodiment, is a subject that is being treated with a compound having epileptogenic potential, e.g., psychotropic drug, or a subject having a disease associated with a risk of developing epilepsy, e.g., autism, can commence a treatment regimen with a compound of formula (I) or formula (II) as described herein.
In another embodiment, the treatment regimen with the compounds of the present invention can commence before any damage or injury to the nervous system has occurred but at a time when such damage or injury can be expected or is likely to occur. For example, such a treatment regimen can begin before a subject undergoes a neurosurgical procedure or is likely to suffer other forms or head or brain trauma, e.g., combat, violent sports or racing, recurrent strokes, TIA's etc.
In another embodiment, the compounds as described herein can be administered daily for a set period of time (week, month, year) after occurrence of the brain damaging injury or initial insult. An attendant physician will know how to determine that the compound of formula (I) or formula (II) as described herein has reached a therapeutically effective level, e.g., clinical exam of a patient, or by measuring drug levels in the blood or cerebro-spinal fluid. One of skill in the art would be able to determine the maximum tolerable dose by means of a physical examination to determine the presence and severity of side effects such as slurred speech, lethargy or impaired coordination.

The present invention further comprises pharmaceutical compositions containing one or more compounds of formula (I) and / or formula (II) with a pharmaceutically acceptable carrier. Pharmaceutical compositions containing one or more of the compounds of the invention described herein as the active ingredient can be prepared by intimately mixing the compound or compounds with a pharmaceutical carrier according to conventional pharmaceutical compounding techniques. The carrier may take a wide variety of forms depending upon the desired route of administration (e.g., oral, parenteral). Thus for liquid oral preparations such as suspensions, elixirs and solutions, suitable carriers and additives include water, glycols, oils, alcohols, flavoring agents, preservatives, stabilizers, coloring agents and the like; for solid oral preparations, such as powders, capsules and tablets, suitable carriers and additives include starches, sugars, diluents, granulating agents, lubricants, binders, disintegrating agents and the like. Solid oral preparations may also be coated with substances such as sugars or be enteric-coated so as to modulate major site of absorption. For parenteral administration, the carrier will usually consist of sterile water and other ingredients may be added to increase solubility or preservation. Injectable suspensions or solutions may also be prepared utilizing aqueous carriers along with appropriate additives.

To prepare the pharmaceutical compositions of this invention, one or more compounds of the present invention as the active ingredient is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules, caplets, gelcaps and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 0.1-1000 mg and may be given at a dosage of from about 0.01-200.0 mg/kg/day, preferably from about 0.1 to 100 mg/kg/day, more preferably from about 0.5-50 mg/kg/day, more preferably from about 1.0-25.0 mg/kg/day or any range therein. The dosages, however, may be varied depending upon the requirement of the patients, the severity of the condition being treated and the compound being employed. The use of either daily administration or post-periodic dosing may be employed.

Preferably these compositions are in unit dosage forms from such as tablets, pills, capsules, powders, granules, sterile parenteral solutions or suspensions, metered aerosol or liquid sprays, drops, ampoules, autoinjector devices or suppositories; for oral parenteral, intranasal, sublingual or rectal administration, or for administration by inhalation or insufflation. Alternatively, the composition may be presented in a form suitable for once-weekly or once-monthly administration; for example, an insoluble salt of the active compound, such as the decanoate salt, may be adapted to provide a depot preparation for intramuscular injection. For preparing solid compositions such as tablets, the principal active ingredient is mixed with a pharmaceutical carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to form a solid preformulation composition containing a homogeneous mixture of a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from 0.01 to about 1000 mg of the active ingredient of the present invention. The tablets or pills of the novel composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

The liquid forms in which the novel compositions of the present invention may be incorporated for administration orally or by injection include, aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable dispersing or suspending agents for aqueous suspensions, include synthetic and natural gums such as tragacanth, acacia, alginate, dextran, sodium carboxymethylcellulose, methylcellulose, polyvinyl-pyrrolidone or gelatin.

The method of treating depression described in the present invention may also be carried out using a pharmaceutical composition comprising any of the compounds as defined herein and a pharmaceutically acceptable carrier. The pharmaceutical composition may contain between about 0.1 mg and 1000 mg, preferably about 50 to 500 mg, of the compound, and may be constituted into any form suitable for the mode of administration selected. Carriers include necessary and inert pharmaceutical excipients, including, but not limited to, binders, suspending agents, lubricants, flavorants, sweeteners, preservatives, dyes, and coatings. Compositions suitable-for oral administration include solid forms, such as pills, tablets, caplets, capsules (each including immediate release, timed release and sustained release formulations), granules, and powders, and liquid forms, such as solutions, syrups, elixers, emulsions, and suspensions. Forms useful for parenteral administration include sterile solutions, emulsions and suspensions.

Advantageously, compounds of the present invention may be administered in a single daily dose, or the total daily dosage may be administered in divided doses of two, three or four times daily. Furthermore, compounds for the present invention can be administered in intranasal form via topical use of suitable intranasal vehicles, or via transdermal skin patches well known to those of ordinary skill in that art. To be administered in the form of a transdermal delivery system, the dosage administration will, of course, be continuous rather than intermittent throughout the dosage regimen.

For instance, for oral administration in the form of a tablet or capsule, the active drug component can be combined with an oral, non-toxic pharmaceutically acceptable inert carrier such as ethanol, glycerol, water and the like. Moreover when desired or necessary, suitable binders; lubricants, disintegrating agents and coloring agents can also be incorporated into the mixture. Suitable binders include, without limitation, starch, gelatin, natural sugars such as glucose or betalactose, corn sweeteners, natural and synthetic gums such as acacia, tragacanth or sodium oleate, sodium stearate, magnesium stearate, sodium benzoate, sodium acetate, sodium chloride and the like. Disintegrators include, without limitation, starch, methyl cellulose, agar, bentonite, xanthan gum and the like.

The liquid forms in suitably flavored suspending or dispersing agents such as the synthetic and natural gums, for example, tragacanth, acacia, methylcellulose and the like. For parenteral administration, sterile suspensions and solutions are desired. Isotonic preparations which generally contain suitable preservatives are employed when intravenous administration is desired.

Compounds of this invention may be administered in any of the foregoing compositions and according to dosage regimens established in the art whenever treatment of depression is required.

The daily dosage of the products may be varied over a wide range from 0.01 to 200 mg / kg per adult human per day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200. 250, 500 and 1000 milligrams of the active ingredient for the symptomatic adjustment of the dosage to the patient to be treated. An effective amount of the drug is ordinarily supplied at a dosage level of from about 0.01 mg/kg to about 1500 mg/kg of body weight per day. Preferably, the range is from about 0.1 to about 100.0 mg/kg of body weight per day, more preferably, from about 0.5 mg/kg to about 50 mg/kg, more preferably, from about 1.0 to about 25.0 mg/kg of body weight per day. The compounds may be administered on a regimen of 1 to 4 times per day.
One skilled in the art will recognize that a therapeutically effective dosage of the compounds of the present invention can include repeated doses within a prolonged treatment regimen that will yield clinically significant results to prevent, reverse, arrest, or inhibit the epileptogenesis.

One skilled in the art will recognize that, both *in vivo* and *in vitro* trials using suitable, known and generally accepted cell and / or animal models are predictive of the ability of a test compound to treat or prevent a given disorder. One skilled in the art will further recognize that human clinical trails including first-in-human, dose ranging and efficacy trials, in healthy patients and / or those suffering from a given disorder, may be completed according to methods well known in the clinical and medical arts.

Determination of effective dosages is typically based on animal model studies followed up by human clinical trials and is guided by determining effective dosages and administration protocols that significantly reduce the occurrence or severity of targeted exposure symptoms or conditions in the subject. Suitable models in this regard includes, for example, murine, rat, porcine, feline, non-human primate, and other accepted animal model subjects known in the art. Alternatively, effective dosages can be determined using in vitro models (e.g., immunologic and histopathologic assays). Using such models, only ordinary calculations and adjustments are typically required to determine an appropriate concentration and dose to administer a therapeutically effective amount of the biologically active agent(s) (e.g., amounts that are intranasally effective, transdermally effective, intravenously effective, or intramuscularly effective to elicit a desired response).

The following Examples are set forth to aid in the understanding of the invention.

### Example 1

### ((3,4-Dihydro-2H-benzo[b][1,4]dioxepin-3-yl)methyl)sulfamide (Compound #3)

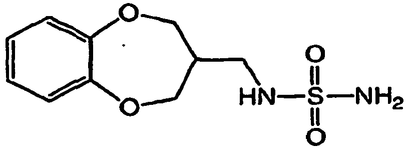

Catechol (5.09 g, 46.2 mmol) and potassium carbonate were combined in acetonitrile and heated to reflux for one hour. 2-Chloromethyl-3-chloro-1-propene (5.78 g, 46.2 mmol) was added and the reaction was continued at reflux for 24 hours. The solution was cooled to room temperature and filtered. The filtrate was evaporated and the residue was diluted with water and extracted with diethyl ether (3 x). The combined organic solution was dried over MgSO₄ and concentrated. Chromatography (2% ethyl ether in hexane) yielded 3-methylene-3,4-dihydro-2H-benzo[b][1,4]dioxepine as a colorless oil.
MS (ESI): 163.2 (M+H⁺)
¹H NMR (300 MHz, CDCl₃), δ: 6.94 (m, 4H), 5.07 (s, 2H), 4.76 (s, 4H).
3-Methylene-3,4-dihydro-2H-benzo[b][1,4]dioxepine (5.00 g, 30.8 mmol) was dissolved in dry THF (100 mL). Borane-THF (1.0 M in THF, 10.3 mL) was added at 0°C. The reaction was stirred at RT for 5 hours. Aminosulfonic acid (6.97 g, 61.6 mmol) was added. The reaction was heated to reflux overnight. The reaction was cooled to room temperature and aqueous sodium hydroxide (3.0 M, 100 mL) was added. The solution was extracted with ethyl acetate (3 x 100 mL). The combined organic solution was dried over MgSO₄. The solution was concentrated under vacuum and purified by chromatography (2% to 8% methanol in dichloromethane) to yield ((3,4-dihydro-2H-benzo[b][1,4]dioxepin-3-yl)methyl)amine as a colorless oil.
MS (ESI): 180.1 (M+H⁺)
¹H NMR (300 MHz, DMSO), δ: 6.92 (m, 4H), 4.21 (m, 2H), 4.07 (m, 2H), 3.33 (broad, 2H), 3.16 (d, *J* = 4 Hz, 1 H), 2.72 (d, *J* = 4 Hz, 1 H), 2.30 (m, 1H).
((3,4-Dihydro-2H-benzo[b][1,4]dioxepin-3-yl)methyl)amine (2.90 g, 16.2 mmol) and sulfamide (3.11 g, 32.4 mmol) were combined in dry dioxane (60 ml) and heated to reflux overnight. Chloroform was added and the precipitate was removed by filtration. The filtrate was concentrated under vacuum and purified by chromatography (2% to 8% acetone in dichloromethane) to yield the title compound as an off-white solid.
258.8 (M+H⁺)
¹H NMR (300 MHz, DMSO), δ: 6.92 (m, 4H), 6.71 (broad, 1H), 6.59 (broad, 2H), 4.19 (m, 2H), 4.04 (m, 2H), 3.00 (m, 2H), 2.39 (m, 1H).

### Example 2

### N-(2,3-Dihydro-benzo[1,4]dioxin-2-ylmethyl)-sulfamide (Compound #1)

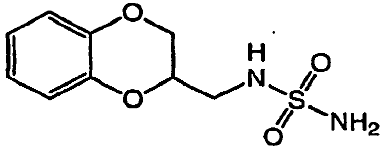

Racemic 2,3-dihydro-1,4-benzdioxin-2-ylmethylamine (4.4 g, 26 mmol) and sulfamide (5.1 g, 53 mmol) were combined in 1,4 dioxane (100 mL) and refluxed for 2 h. The reaction was cooled to room temperature and a small amount of solid was filtered and discarded. The filtrate was evaporated in vacuo and the residue was purified using flash column chromatography (DCM:Methanol - 10:1) to yield a white solid. The solid was recrystallized from DCM to yield the title compound as a white solid.
mp: 97.5 - 98.5°C
Elemental Analysis:
Anal Calc: C, 44.25; H, 4.95; N, 11.47; S, 13.13
Anal Found: C, 44.28; H, 4.66; N, 11.21; S, 13.15
H¹ NMR (DMSO d6) 8 6.85 (m, 4H), 6.68 (bd s, 3H, NH), 4.28 (m, 2H), 3.97 (dd, J = 6.9, 11.4 Hz, 1H), 3.20 (m, 1 H), 3.10 (m, 1H).

### Example 3

### (Benzo[1,3]dioxol-2-ylmethyl)sulfamide (Compound #2)

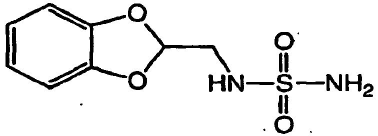

Catechol (10.26 g, 93.2 mmol), sodium methoxide (25% by weight in methanol, 40.3 g, 186 mmol), and methyl dichloroacetate (13.3 g, 93.2 mmol) were combined in dry methanol (100 mL). The solution was heated to reflux overnight. The reaction was cooled to room temperature, acidified by addition of concentrated hydrochloric acid and then reduced in volume under vacuum to about 50 mL. Water was added and the mixture was extracted with diethyl ether (3 x 100 mL). The combined organic solution was dried with MgSO₄, concentrated to a brown solid, and chromatographed (2% ethyl acetate in hexane) to yield benzo[1,3]dioxole-2-carboxylic acid methyl ester as a colorless oil.
MS (ESI): 195.10 (M+H⁺).
¹H NMR (300 MHz, CDCl₃), δ: 6.89 (broad, 4H), 6.29 (s, 1H), 4.34 (q, *J* =7 Hz, 2H), 1.33 (t, *J* =7 Hz, 3H).
To benzo[1,3]dioxole-2-carboxylic acid methyl ester (7.21 g, 40.0 mmol) was added ammonium hydroxide (29% in water, 10 mL) and enough acetonitrile to make the mixture homogeneous (-5 mL). The solution was stirred for two hours at room temperature and then distilled water was added. Benzo[1,3]dioxole-2-carboxylic acid amide precipitated as a white solid and was collected by filtration and used without further purification.
MS (ESI): 160.00 (M+H⁺)
¹H NMR (300 MHz, DMSO), δ: 7.99 (s, broad, 1H), 7.72 (s, broad, 1 H), 6.94 (m, 2H) 6.86 (m, 2H), 6.30 (s, 1 H).
Benzo[1,3]dioxole-2-carboxylic acid amide (5.44 g, 32.9 mmol) was dissolved in tetrahydrofuran (THF, 100 mL). Lithium aluminum hydride (LAH, 1 M in THF, 39.5 mL), 39.5 mmol) was added slowly to the solution at room temperature. The reaction was stirred at room temperature for 24 hours. Distilled water was added to destroy the excess LAH. Aqueous sodium hydroxide (3.0 M, 100 mL) was added and the solution was extracted with ethyl acetate (3 x 100 mL). The combined organic solution was washed with water and dried over MgSO₄. The solvent was evaporated to yield C-benzo[1,3]dioxol-2-yl-methylamine as a colorless oil.
MS (ESI): 152:1 (M+H⁺)
¹ H NMR (300 MHz, CDCl₃), δ: 6.87 (m, 4H), 6.09 (t, *J* = 4 Hz, 1 H), 3.13 (d, *J* = 4 Hz, 2H)
C-Benzo[1,3]dioxol-2-yl-methylamine (2.94 g, 19.4 mmol) and sulfamide (3.74 g, 38.9 mmol) were combined in dry dioxane (50 mL) and the solution was heated to reflux overnight. The reaction was concentrated and the residue was chromatographed (2% to 10% acetone in dichloromethane) to yield the title compound as a white solid.
MS (ESI): 230.0 (M+H⁺)
¹H NMR (300 MHz, CDCl₃). δ: 6.87 (m, 4H), 6.25 (t, *J* = 4 Hz, 1H), 4.79 (broad, 1 H), 4.62 (broad, 1 H), 3.64 (d, *J* = 4 Hz, 2H).

### Example 4

### (2S)-(-)-N-(2,3-Dihydro-benzo[1,4]dioxin-2-ylmethyl)-sulfamide (Compound #4)

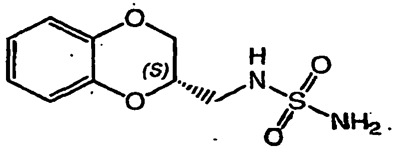

Catechol (13.2 g, 0.12 mol) and potassium carbonate (16.6 g, 0.12 mol) were stirred in DMF (250 mL) and (2R)-glycidyl tosylate (22.8 g, 0.10 mol) was added and the reaction was stirred at 60°C for 24 h. The reaction was cooled to room temperature and diluted with ice water (1 L) and extracted with diethyl ether (4 times). The combined organic solution was washed 3 times with 10% potassium carbonate, once with water, once with brine and evaporated in vacuo to yield a white solid which was purified by flash column chromatography (DCM:Methanol - 50:1) to yield ((2S)-2,3-dihydro-benzo[1,4]dioxin-2-yl)-methanol as a solid.
The solid (13.3 g, 68 mmol) was dissolved in pyridine (85 mL) cooled to 0°C, p-toluenesulfonyl chloride (13.0 g, 68 mmol) was added and the reaction mixture stirred at room temperature for 20h. The reaction was diluted with diethyl ether (1 L) and 1 N HCl (1.2 L). The organic layer was separated and washed 2 times with 1 N HCl (500 mL), 4 times with water (150 mL), once with brine, dried (MgSO₄) and evaporated in vacuo to yield a white solid which was purified by flash column chromatography (HeptEA -2:1) to yield toluene-4-sulfonic acid (2S)-2,3-dihydro-benzo[1,4]dioxin-2-ylmethyl ester as a white solid.
The white solid was combined with potassium phthalimide (14.4 g, 78 mmol) in DMF (250 mL) and heated to reflux for 1 h, cooled to room temperature and poured into vigorously stirring water (1.5 L) and stirred 30 min. White solid was filtered and the solid was washed several times with water, 2% NaOH, and water again and let air dry to yield a (2S)-2-(2,3-Dihydrobenzo[1,4]dioxin-2-ylmethyl)-isoindote-1,3-dione as white powdery solid.
The powdery white solid was combined with hydrazine (2.75 g, 86 mmol) in EtOH (225 mL) and heated at reflux for 2 h, cooled to room temperature and 1 N HCl added to pH 1.0 and stirred for 15 min. White solid was filtered and washed with fresh EtOH (solid discarded) and the filtrate was evaporated in vacuo to a solid, which was partitioned between diethyl ether and dilute aqueous NaOH. The diethyl ether solution was dried (Na₂SO₄) and evaporated in vacuo to a yield a light yellow oil. The oil was purified by flash column chromatography (DCM:MeOH - 10:1) to yield an oil. A portion of the oil (4.82 g, 29 mmol) in 2-propanol (250 mL) was treated with 1 N HCl (30 mL) and heated on steambath until homogeneous and then let cool to room temperature. After 3 h, the mixture was ice cooled for 2 h. A white flaky solid (the corresponding HCl salt of (2S)-C-(2,3-Dihydro-benzo[1,4]dioxin-2-yl)-methylamine) was filtered off and then recrystallized again from 2-propanol to yield a white solid.
[α]_{D} = -69.6 (c = 1.06, EtOH)
The white solid was partitioned between DCM and dilute NaOH, and the DCM was dried (NaSO₄) and evaporated in vacuo to yield (2S)-C-(2,3-Dihydrobenzo[1,4]dioxin-2-yl)-methylamine as an oil.
[α]_{D} = -57.8 (c = 1.40, CHCl₃)
The oil (2.1 g, 12.7 mmol) and sulfamide (2.44 g, 25.4 mmol) were refluxed in dioxane (75 mL) for 2 h and the crude product was purified by flash column chromatography (DCM:MeOH 10:1) to yield a white solid, which was recrystallized from DCM to yield the title compound as a white crystalline solid.
mp 102-103°C
[α]_{D} = -45.1 °(c = 1.05, M);
¹H NMR (DMSOd6) δ 6.86 (m, 4H), 6.81 (bd s, 3H, NH), 4.3 (m, 2H), 3.97 (dd, J = 6.9, 11.4 Hz, 1 H), 3.20 (dd, J = 5.5, 13.7 Hz, 1 H), 3.10 (dd, J = 6.9, 13.7 Hz, 1 H)

### Elemental Analysis:

| | |
|---|---|
| Anal Calc: | C, 44.25; H, 4.95; N, 11.47; S, 13.13 |
| Anal Found: | C, 44.20; H, 4.69; N, 11.40; S, 13.22. |

### Example 5

### N-(2,3-Dihydro-benzon[1,4]dioxin-2-ylmethyl)-N',N' dimethylsulfamide (Compound #6)

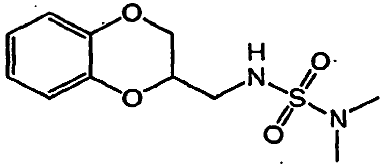

Racemic 2,3-dihydro-1,4-benzdioxin-2-ylmethylamine (8.25 g, 5.0 mmol) and triethylamine (1.52 g, 15 mmol) were combined in DMF (10 mL) and cooled in an ice bath as dimethylsulfamoyl chloride (1.44 g, 10 mmol) was added. The reaction mixture was then stirred for 3 hr with continued cooling. The reaction mixture was partitioned between ethyl acetate and water, and the ethyl acetate solution was washed with brine, dried (MgSO₄) and evaporated in vacuo to yield an oil. The oil was purified using flash column chromatography (ethyl acetate:Heptane - 1:1) to yield a white solid, which was recrystallized (ethyl acetate/Hexane) to yield the title compound as a white floccular solid.
mp 76 - 78°C
MS 273 (MH⁺)
Elemental Analysis:
Anal Calc: C, 48.52; H, 5.92; N, 10.29; S, 11.78
Anal Found: C, 48.63; H, 5.62; N, 10.20; S, 11.90
¹H NMR (CDCl₃) δ 6.87 (m, 4H), 4.59 (bd m, 1 H, NH), 4.35 (m, 1 H), 4.27 (dd, J = 2.3, 11.4 Hz, 1 H), 4.04 (dd, J = 7.0, 11.4, 1 H), 3.36 (m, 2H), 2.82 (s, 6H).

### Example 6

### N-(2,3-Dihydro-benzo[1,4]dioxin-2-ylmethyl)-N-methylsulfamide (Compound #7)

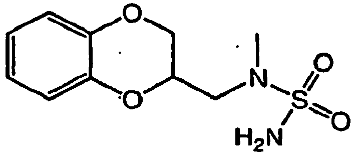

Racemic 2,3-dihydro-1,4-benzdioxin-2-ylmethylamine (825 mg, 5 mmol) was dissolved in ethyl formate (15 mL), refluxed for 30 min and evaporated in vacuo to yield N-(2,3-dihydro-benzo[1,4]dioxin-2-ylmethyl)-formamide as an oil.
The oil in diethyl ether (25 mL) was treated with 1 M LAH in THF (9.0 mL, 9.0 mmol) at 0°C and stirred for 5 h at room temperature. The reaction was cooled in an ice bath and quenched with water (0.50 mL), followed by 3 N NaOH (0.50 mL) and water (0.50 mL). The mixture was then stirred at room temperature for 1 h. Solid was filtered and the filtrate was evaporated in vacuo to yield a residue which was partitioned between 1N HCl and diethyl ether. The aqueous phase was basified with 1N NaOH and extracted with diethyl ether. The organic phase was dried (MgSO₄) and evaporated in vacuo to yield (2,3-dihydro-benzo[1,4]dioxin-2-ylmethyl)-methyl-amine as an oil.
MS 180 (MH⁺)
¹H NMR (CDCl₃) δ 6.85 (m, 4H), 4.30 (m, 2H), 4.02 (dd, J = 7.9, 11.6 Hz, 1 H), 2.85 (m, 2H), 2.50 (s, 3H)
The oil (380 mg, 2.1 mmol) and sulfamide (820 mg, 8.5 mmol) were combined in dioxane (15 mL), refluxed for 1.5 h and evaporated in vacuo to yield a crude residue. The residue was purified via column chromatography (ethyl acetate/Heptane 1:1) and the resultant solid was recrystallized from ethyl acetate/Hexane to yield the title compound as a white solid.
mp 97-98°C
MS 257 (M⁻¹)

### Elemental Analysis:

| | |
|---|---|
| Anal Calc: | C, 46.50; H, 5.46; N, 10.85; S, 12.41 |
| Anal Found: | C, 46.48; H, 5.65; N, 10.90; S, 12.07 |

¹H NMR (CDCl₃) δ 6.86 (m, 4H), 4.52 (bs, 2H), 4.46 (m, 1 H), 4.29 (dd, J = 2.3, 11.5 Hz, 1 H), 4.05 (dd, J = 6.5, 11.5 Hz, 1 H), 3.51 (dd, J = 6.7, 14.9 Hz, 1H), 3.40 (dd, J = 5.9, 14.9 Hz, 1 H), 2.99 (s, 3H).

### Example 7

### (2S)-(-)-N-(6-Chloro-2,3-dihydro-benzol[1,4]dioxin-2-ylmethyl)-sulfamide (Compound #8)

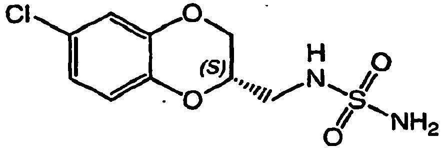

Following the procedure outlined in Example 4 above, 4-chlorocatechol was reacted to yield a mixture of (2S)-C-(7-Chloro-2,3-dihydrobenzo[1,4]dioxin-2-yl)-methylamine and (2S)-C-(6-Chloro-2,3-dihydrobenzo[1,4]dioxin-2-yl)-methylamine (ca. 3:1 ratio of 6-chloro:7-chloro isomers by RP HPLC).
The mixture was dissolved in 2-propanol (100 mL) and 1 N HCl in diethyl ether was added until pH = 1.0 was attained. The hydrochloride salt that precipitated was filtered (2.65 g) and re-crystallized from methanol/IPA to yield white crystals. The white crystals were partitioned between DCM and dilute NaOH. The DCM was dried and evaporated in vacuo to yield purified (2S)-C-(6-Chloro-2,3-dihydro-benzo[1,4]dioxin-2-yl)-methylamine as an oil.
[α]_{D} = -67.8 (c = 1.51, CHCl₃)
The oil (7.75 mmol) and sulfamide (1.50 g, 15.5 mmol) were combined in dioxane (50 mL) and refluxed for 2.0 h, cooled to room temperature and evaporated in vacuo to yield a solid. The product was purified via flash column using DCM/methanol 20:1 to yield the title compound as a white solid.
MS 277 (M⁻¹)
[α]_{D} = -59.9° (c = 1.11, M)
¹H NMR (CDCl₃) δ 6.90 (d, J = 2.2 Hz, 1H), 6.81 (m, 2H), 4.76 (m, 1H), 4.55 (s, 2H), 4.40 (m, 1 H), 4.29 (dd, J = 2.4, 11.5 Hz, 1 H), 4.05 (dd, J = 7.1, 11.5 Hz, 1 H), 3.45 (m, 2H)

### Elemental Analysis:

| | |
|---|---|
| Anal Calc: | C, 38.78; H, 3.98; N, 10.05 |
| Anal Found: | C, 38.80; H, 3.67; N, 9.99. |

The filtrates of the crystallized hydrochloride salt of (2S)-C-(6-Chloro-2,3-dihydro-benzo[1,4]dioxin-2-yl)-methylamine prepared above were recovered (ca. 1:1 of 6-chloro:7-chloro isomers) and evaporated in vacuo to yield a solid, which was partitioned between DCM (200 mL) and dilute NaOH (0.5 M, 50 mL). The DCM solution was washed once with brine, dried (Na₂SO₄) and evaporated in vacuo to yield an oil, which was purified via reverse phase HPLC (10 - 50% ACN with 0.16% TFA in water with 0.20% TFA) to yield (2S)-C-(7-Chloro-2,3-dihydro-benzo[1,4]dioxin-2-yl)-methylamine as a residue.
The residue was combined with sulfamide (0.90 g, 9.4 mmol) in dioxane (25 mL) and refluxed for 2.5 h, cooled to room temperature and evaporated in vacuo to yield an oil. The oil was purified by flash column chromatography using DCM/methanol -10:1 to yield (2S)-(-)-N-(7-Chloro-2,3-dihydrobenzo[1,4]dioxin-2-ylmethyl)-sulfamide as a white solid.
MS 277 (M⁻¹)
¹H NMR (CDCl₃/CD₃OD) δ 6.88 (d, J = 0.7 Hz, 1H), 6.81 (m, 2H), 4.37 (m, 1 H), 4.30 (dd, J = 2.3, 11.6 Hz, 1 H), 4.04 (dd, J = 7.0, 11.6 Hz, 1 H), 3.38 (m, 2H).

### Example 8

### Chroman-2-ylmethylsulfamide (Compound #10)

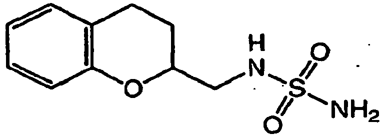

Chroman-2-carboxylic acid (4.5 g, 25 mmol) and HOBT (3.86 g, 25 mmol) were combined in DCM (40 mL) and DMF (10 mL). Dimethylaminopropyl ethylcarbodiimide (EDC, 4.84 g, 25 mmol) was added at room temperature and the reaction mixture was stirred for 30 min. Ammonium hydroxide (2.26 mL, 33.4 mmol) was added and the reaction mixture was stirred for 16h. The reaction mixture was diluted with DCM (50 mL) and water (50 mL) and the pH of the mixture was adjusted to about pH = 3.0 with 1 N HCl. The DCM was separated and the aqueous phase extracted twice with DCM. The combined DCM phase was dried (Na₂SO₄) and evaporated in vacuo to yield an oil, which was purified with flash column chromatography (ethyl acetate) to yield an oil.
The oil (5.35 g, 30 mmol) in THF (90 mL) was stirred as 1 M LAH in THF (36 mL, 36 mmol) was added and the reaction mixture was then stirred at room temperature for 20 h. The reaction was quenched with water, stirred for 2 hours, the solution decanted, dried (Na₂SO₄) and evaporated in vacuo to yield C-chroman-2-yl-methylamine as an oily amine.
The oily amine (1.63 g, 10 mmol) and sulfamide (1.92 g, 20 mmol) were combined in dioxane (50 mL) and brought to reflux for 2 h. The solution was cooled and evaporated in vacuo to yield an oil; which was purified via column chromatography (DCM:Methanol 10:1) to yield a white solid. The solid was recrystallized from ethyl acetate/hexane to yield chroman-2-yimethylsulfamide as a white solid.
mp 100-101 °C
MS 241 (M⁻¹)

### Elemental Analysis:

Anal Calc: C, 49.57; H, 5.82; N, 11.56; S, 13.23
Anal Found: C, 49.57; H, 5.80; N, 11.75; S, 13.33.

### Example 9

### 2-(2,3-Dihydro-benzo[1,4]dioxin-2-yl)-ethylsulfamide (Compound #16)

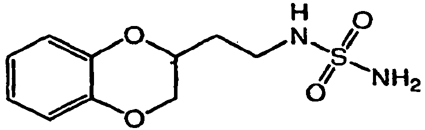

Potassium cyanide (2.05 g, 31.5 mmol) was added to 2-bromomethyl-(2,3 dihydrobenzo[1,4]dioxine) (6.87 g, 30 mmol) in DMSO (90 mL) and stirred at ambient temperature for 20 h. The reaction mixture was then diluted with water (250 mL) and extracted twice with diethyl ether. The diethyl ether was washed with water, then washed twice with brine, dried (Na₂SO₄) and evaporated in vacuo to yield 2-cyanomethyl-(2,3 dihydrobenzo[1,4]dioxine) as a white solid.
¹H NMR (CDCl₃) δ 6.89 (m, 4H), 4.50 (m, 1 H), 4.31 (dd, J = 2.3, 11.5 Hz, 1H), 4.08 (dd, J = 6.2, 11.6 Hz, 1 H), 2.78 (d, J = 6.1, Hz, 2H)
The 2-cyanomethyl-(2,3 dihydrobenzo[1,4]dioxine) was dissolved in THF (50 mL) and 1 M BH₃ in THF (80 mL, 80 mmol) was added and the reaction mixture refluxed for 5 h, then stirred at ambient temperature for 16h. With ice bath cooling, 2N HCl was added until pH = 1.0 was achieved. The reaction mixture was then stirred for 1 h at room temperature and evaporated in vacuo to yield an oil. The oil was partitioned between 3N NaOH and diethyl ether, and the diethyl ether solution was washed with brine, dried (Na₂SO₄) and evaporated in vacuo to yield crude 2-(2,3 dihydrobenzo[1,4]dioxin-2-yl)ethlamine.
MS (M+H)⁺ 180.
The crude 2-(2,3 dihydrobenzo[1,4]dioxin-2-yl)ethylamine in dioxane (100 mL) was combined with sulfamide (3.0 g, 31 mmol)) and heated to reflux for 2 h. The solution was cooled and evaporated in vacuo to yield an orange solid, which was purified by column chromatography (DCM:MeOH - 10:1) to yield a white solid. The solid was re-crystallized from DCM to yield the title compound as a solid.
MS (M-1) 257
MP 101 - 103°C (corr)
¹H NMR (CDCl₃): δ 6.86 (m, 4H), 4.70 (m, 1H), 4.52 (s, 2H), 4.30 (m, 2H), 3.94 (dd, J = 7.4, 11.3 Hz, 1H), 3.43 (dd, J = 6.4, 12.9 Hz, 2H), 1.94 (dd, J = 6.5, 12.9, 2H).

### Elemental Analysis:

| | |
|---|---|
| Measured: | C, 46.48; H, 5.60; N, 10.81; S, 12.41 |
| Calculated: | C, 46.50; H, 5.46; N, 10.85; S, 12.41 |

### Example 10

### (2S)-(-)-N-(6,7 Dichloro-2,3-dihydro-benzo[1,4]dioxin-2-ylmethyl)sulfamide (Compound #29)

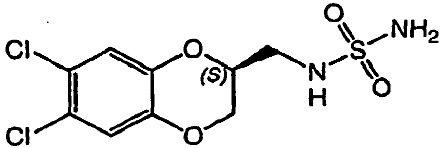

4,5 Dichloroatechol (8.6 g, 48 mmol) and potassium carbonate (6.64 g, 48 mmol) were stirred in DMF (200 mL). (2R)-Glycidyl tosylate (9.12 g, 40 mmol) was added and the reaction mixture was stirred at 60°C for 24 h. The reaction mixture was cooled to room temperature and then diluted with ice water (600 mL) and extracted with diethyl ether (4 times). The combined organic solution was washed 3 times with 10% potassium carbonate, twice with brine, dried (MgSO₄) and evaporated in vacuo to yield a viscous oil of (2S)-2-(6,7-dichloro-2,3-dihydro-benzo[1,4]dioxine) methanol.
The (2S)-2-(6,7 dichloro-2,3-dihydro-benzo[1,4]dioxine) methanol oil (6.4 g, 27 mmol) was dissolved in pyridine (50 mL) cooled to 0°C. Then, p-toluenesulfonyl chloride (5.2 g, 27 mmol) was added and the reaction mixture was stirred at room temperature for 20h. The reaction mixture was diluted with diethyl ether and 1 N HCl (750 mL) and the organic layer was separated and washed 2 times with 1 N HCl (250 mL), once with water (150 mL), twice with brine, dried (MgSO₄) and evaporated in vacuo to yield light yellow solid of toluene-4-sulfonic acid (2S)-6,7-dichloro-2,3-dihydro-benzo[1,4]dioxin-2-ylmethyl ester.
¹H NMR (CDCl3): δ 7.79 (d, J = 8.3 Hz, 2H), 7.36 (d, J = 8.0 Hz, 2H), 6.94 (s, 1H), 6.83 (s, 1H), 4.37 (m, 1H), 4.2 (m, 3H); 4.03 (dd, J = 6.3, 11.7 Hz, 1H), 2.47 (s, 3H).
Toluene-4-sulfonic acid (2S)-6,7-dichloro-2,3-dihydro-benzo[1,4]dioxin-2-ylmethyl ester (8.0 g, 20.5 mmol) was combined with potassium phthalimide (6.1 g, 33 mmol) in DMF (75 mL) and heated to reflux for 1 h, cooled to room temperature and poured into vigorously stirring water (0.5 L) and then stirred 30 min. White solid was filtered and the solid was washed several times with water, 2% NaOH, and water again and then let air dry to yield (2S)-2-(6,7-dichloro-2,3-dihydro-benzo[1,4]dioxin-2-ylmethyl)-isoindofe-1,3-dione (6.0 g, 80%) as a white powdery solid.
The white powdery solid was combined with hydrazine (1.06 g, 33 mmol) in EtOH (80 mL) and heated at reflux for 2 h, then cooled to room temperature. 1 N HCl was added to adjust the reaction mixture's pH to pH 1.0 and the reaction mixture was then stirred for 15 min. White solid was filtered and washed with fresh EtOH (solid discarded) and the filtrate was evaporated in vacuo to a solid, which was partitioned between diethyl ether and dilute aqueous NaOH. The diethyl ether solution was dried (Na₂SO₄) and evaporated in vacuo to a yield a viscous oil of (2S)-2-aminomethyl-(6,7-dichloro-2,3-dihydro-benzo[1,4]dioxine).
¹H NMR (CDCl3): δ 6.98 (s, 1 H), 6.96 (s, 1H), 4.25 (dd, J = 2.0, 11.2 Hz, 1H), 4.15 (m, 1 H), 4.0 (m, 1H), 2.97 (d, J = 5.5 Hz, 2H)
A portion of the oil (3.8 g, 16 mmol) and sulfamide (3.1 g, 32.4 mmol) were refluxed in dioxane (100 mL) for 2 h and the crude product was purified by flash column chromatography (DCM:MeOH 20:1) to yield the title compound as a white solid, which was recrystallized from ethyl acetate / hexane to yield the title compound as a white crystalline solid.
MS [M-H]⁻ 311.0
mp 119-121 °C
[α]_{D} = -53.4 ° (c = 1.17, M)
¹H NMR (DMSOd6): δ 7.22 (s, 1 H), 7.20 (s, 1 H), 6.91 (bd·s, 1H), 6.68 (bd s, 2H), 4.35 (m, 2H), 4.05 (dd, J = 6.5, 11.5 Hz, 1H), 3.15 (m, 2H) Elemental Analysis:

### Elemental Analysis:

| | |
|---|---|
| Measured: | C, 34.52; H, 3.22; N, 8.95; Cl, 22.64; S, 10.24 |
| Calculated: | C, 34.64; H, 2.68; N, 8.87; Cl, 22.94; S, 10.35. |

### Example 11

### (2S)-(-)-N-(7-Amino-2,3-dihydro-benzo[1,4]dioxin-2-ylmethyl)-sulfamide (Compound #36)

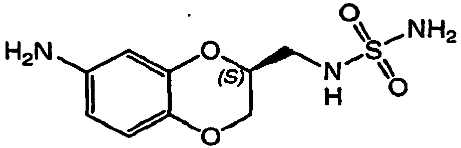

(2S)-(-)-N-(2,3-Dihydro-7-nitro-benzo[1,4]dioxin-2-ylmethyl)-sulfamide (1.2 g, 4.15 mmol), was prepared from 4-nitrocatechol according to the process outlined in Example 4. The (2S)-(-)-N-(2,3-Dihydro-7-nitro-benzo[1,4]dioxin-2-ylmethyl)-sulfamide, was then combined with 10% Pd/C in methanol (120 mL) and shaken under hydrogen atmosphere (39 psi) at room temperature for 3 h. The solids were filtered and washed with 10% M in DCM and the filtrate was evaporated in vacuo to yield crude product. The crude product was dissolved in 0.2 N HCl (25 mL), frozen and lyophilized to yield the title compound as a white flaky solid, as the corresponding hydrochloride salt.
MS (M+H)⁺ 260
¹H NMR (DMSO d6): δ 10.2 (bd s, 3H), 6.86 (m, 1H), 6.85 (s, 1 H), 6.74 (dd, J = 2.5, 8.4 Hz, 1 H), 4.22 (m, 2H), 3.88 (dd, J = 6.7, 11.4 Hz, 1 H), 3.04 (m, 2H)

### Example 12

### (2S)-(-)-N-(7-Methyl-2,3-dihydro-benzo[1,4]dioxin-2-ylmethyl)-sulfamide (Compound #19)

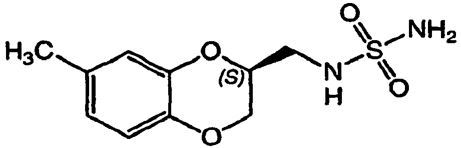

Title compound was prepared according to the procedure described in Example 4 above, starting with 4-methylcatechol, to yield a white solid, which was recrystallized from ethyl acetate/ hexane to yield the title compound as a white solid.
MS [M-H]⁻ 257
¹H NMR (CDCl3): δ 6.76 (m, 1H), 6.66 (m, 2H), 4.80 (m, 1H), 4.57 (bd s, 1H), 4.40 (m, 1H), 4.28 (m, 1 H), 4.03 (dd, J = 6.9, 11.4 Hz, 1H), 3.45 (m, 2H), 2.25 (s, 3H).

### Elemental Analysis

| | |
|---|---|
| Calculated: | C, 46.50; H, 5.46; N, 10.85; S, 12.41 |
| Found: | C, 46.65; H, 5.60; N, 10.84; S, 12.61. |

### Example 13

### In Vivo, Amygdala Kindling Assay

The development of amygdala kindling is an established model of epileptogenesis. (Amano K, Hamada K; Yagi K, Seino M. Antiepileptic effects of topiramate on amygdaloid kindling in rats. Epilepsy Res. 1998 Jul;31(2):123-8; Barton ME, White HS. The effect of CGX-1007 and Cl-1041, novel NMDA receptor antagonists, on kindling acquisition and expression. Epilepsy Res. 2004 Mar;59(1):1-12; Loscher W, Honack D, Rundfeldt C. Antiepileptogenic effects of the novel anticonvulsant levetiracetam (ucb L059)in the kindling model of temporal lobe epilepsy. J Pharmacol Exp Ther. 1998 Feb;284(2):474-9; McNamara JO. Analyses of the molecular basis of kindling development. Psychiatry Clin Neurosci. 1995 June; 49(3):S175-8. Review. ; Morimoto K, Katayama K, Inoue K, Sato K. Effects of competitive and noncompetitive NMDA receptor antagonists on kindling and LTP. Pharmacol Biochem Behav. 1991 Dec;40(4):893-9; Racine, R.J. 1972. Modification of seizure activity by electrical stimulation. II. Motor seizure. Electroencephalogr. Clin. Neurophysiol. 32, 281-294). The kindled rat model of partial epilepsy is an accepted model of epileptogenesis (McNamara JO. Analyses of the molecular basis of kindling development. Psychiatry Clin Neurosci. 1995 Jun;49(3):S175-8. Review.).

Briefly, the assay procedure was as follows. Adult male, Sprague-Dawley rats weighing between 250-300 g were obtained from Charles River, Wilmington, MA. All the animals were housed on a 12:12 light dark cycle and permitted free access to both food (Prolab RMH 3000) and water except when removed from the home cage for experimental procedures. Animals were cared for in a matter consistent with the recommendations detailed in the National Research Council Publication, "Guide for the Care and Use of Laboratory Animals" in a temperature controlled, pesticide-free facility. Kindling stimulations were routinely performed between 9 AM - 2 PM to avoid any circadian variations.
Compound #8 was triturated in a small volume of 0.5% methylcellulose, sonicated for 10 min, and brought to a final volume with 0.5% methylcellulose. Compound #8 was administered systemically (i.p.) in a volume of 0.04 ml/10 g body weight and all tests were conducted at the pre-determined time of peak effect of 0.5 hours after i.p. administration.

The ability of Compound #8 to block the expression of amygdala kindled seizures was determined as follows. Rats were anaesthetized with a ketamine (120mg/kg; i.p.) and xylazine (12mg/kg, i.p.) cocktail. Under aseptic conditions, a bipolar electrode (Plastic One, Roanoke, VA) was stereotaxically implanted into the right basolateral amygdala (AP -2.2, ML -4.7, DV -8.7; Paxinos and Watson). Anterior-posterior and lateral measurements were from Bregma, whereas the dorsal-ventral measurement was from the skull surface. Sterile skull screws (3-4) were implanted for the indifferent reference electrode Electrodes were fixed using dental cement and acrylic. The wound was then closed using sterile 18/8 Michel suture clips (Roboz, Gaithersburg, MD). Neomycin antibiotic ointment was applied to the wound and a single dose of penicillin (60,000 IU, *im,* AgriLabs) was administered to the each rat before returning them to clean cages for one week of post operative recovery
Amygdala kindling was then performed according to the following protocol. Following a brief acclimation (< 5 minutes) to the recording chamber, baseline EEG recordings were obtained (MP 100, Biopac Systems Inc., Goleta, CA). Rats were then randomized to receive either vehicle (0.5% methylcellulose) or Compound #8 (75 mg/kg, i.p.) (n = 10 rats per group). On the day of the experiment, a single dose of Compound #8 or 0.5% methylcellulose was administered 30 minutes prior to amygdala stimulation (200 µA for 2 seconds). The behavioral seizure score and AD duration was recorded for rats in each treatment group. Behavioral seizure scores were determined using the Racine scale; i.e., 0 = no response; stage 1 = grooming/hyperactivity; stage 2 = head nodding/tremor; stage 3 = unilateral forelimb clonus; stage 4 = clonus with rearing; and stage 5 = generalized tonic-clonic seizure with rearing and falling (Racine, 1972). After-discharge (AD) activity was digitally recorded for up to 180 seconds following the stimulation train and the duration of the primary AD was measured. Rats were considered fully kindled when they displayed five consecutive Stage 4 or 5 generalized seizures. Daily stimulations were continued for up to 13 consecutive days in all three groups until rats in the vehicle-treated group were fully kindled (i.e., five consecutive Stage 4 or 5 seizures). At this time, all rats were allowed a one-week stimulus and drug-free period; after which they were re-challenged in the absence of drug with the same stimulus employed during the acquisition phase (i.e., days 1-13). Rats treated with Compound #8 were subsequently stimulated once per day until they reached a fully kindled state.

The after-discharge (AD) duration in both the vehicle and Compound #8 treated groups displayed a progressive increase over the course of the kindling acquisition phase. No statistical difference between treatment groups was observed.
Compound #8 prevented the acquisition of the full generalized kindled seizure. This conclusion is based on the finding that the seizure score at the conclusion of the drug- and stimulus-free period remained significantly lower than that of the rats in the vehicle-treated group (Compound #8 = 1.4 ± 0.40 vs. vehicle = 4.6 ± 0.24). Additionally, when stimulated in the absence of drug, the seizure score of rats in the Compound #8 treatment group increased at a rate that was parallel to that observed in the vehicle-treated rats - supporting the conclusion that Compound #8 delayed the acquisition of kindling by several days.

The results of this study demonstrate that Compound #8 possesses the ability to modify the development of kindling in the amygdala kindled rat model of partial epilepsy. These results are consistent with the conclusion that Compound #8 possesses disease-modifying effects. This conclusion is based on the finding that the seizure score, at the conclusion of the drug- and stimulation-free period, of rats in the Compound #8 treatment group remained significantly lower than that of the vehicle-treated rats. Furthermore, once the stimulation protocol was resumed in the absence of drug, the seizure score progressed at a rate that was parallel to the vehicle-treated group.
The finding that the seizure score, but not the after-discharge duration (ADD), in the compound treatment group one-week after the stimulus- and drug-free week was markedly lower than that of the vehicle-treated group suggesting that Compound #8 prevented the acquisition of the secondarily generalized seizure but not the focal seizure.

### Example 14

As a specific embodiment of an oral composition, 100 mg of the Compound #8 prepared as in Example 7 is formulated with sufficient finely divided lactose to provide a total amount of 580 to 590 mg to fill a size O hard gel capsule.

## Claims

1. A compound of formula (I) 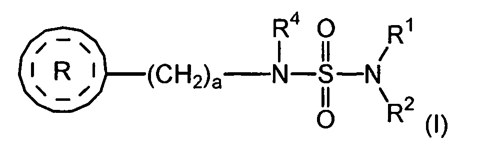 or a pharmaceutically acceptable salt thereof for use in the treatment, prevention, arrest, inhibition or reversal of epileptogenesis, wherein said treatment comprises administering said compound or pharmaceutically acceptable salt thereof to a patient in need of treatment with an anti-epileptogenic drug (an AEGD), wherein said patient is at risk for the development of epilepsy, a seizure disorder or an analogous seizure-related disorder,
wherein
R¹ and R² are each independently selected from the group consisting of hydrogen and lower alkyl;
R⁴ is selected from the group consisting of hydrogen and lower alkyl;
a is an integer from 1 to 2; 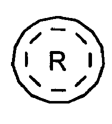 is selected from the group consisting of 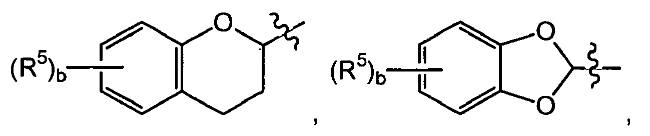 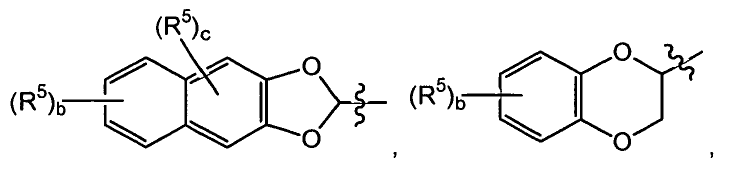 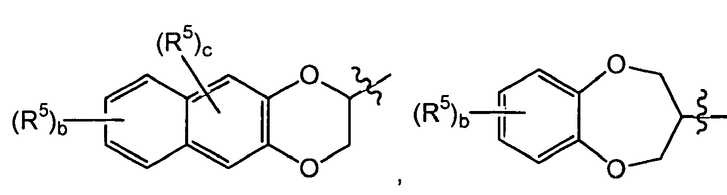 and 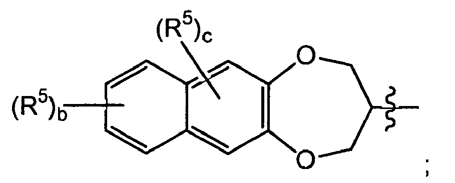 wherein b is an integer from 0 to 4; and wherein c is an integer from 0 to 2;
each R⁵ is independently selected from the group consisting of halogen, lower alkyl and nitro;
wherein "lower alkyl" refers to a carbon chain composition of 1-4 carbon atoms,
provided that when 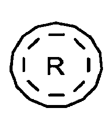 is 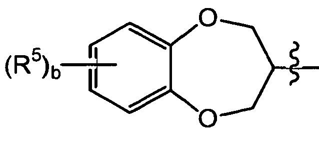 or 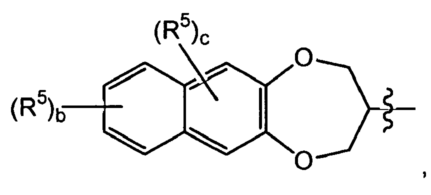 then a is 1.

2. The compound or a pharmaceutically acceptable salt thereof as in Claim 1 for use according to claim 1, wherein
R¹ and R² are each independently selected from the group consisting of hydrogen and lower alkyl;
R⁴ is selected from the group consisting of hydrogen and lower alkyl;
a is an integer from 1 to 2; 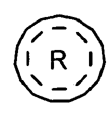 is selected from the group consisting of 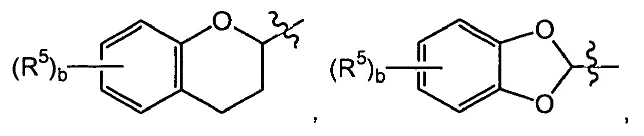 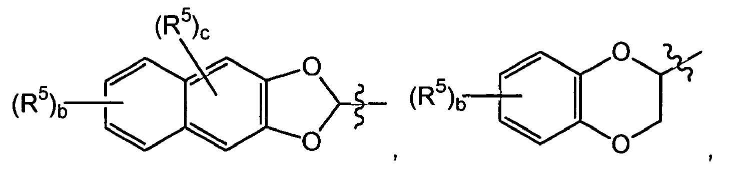 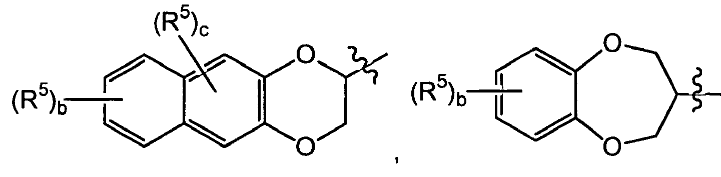 and 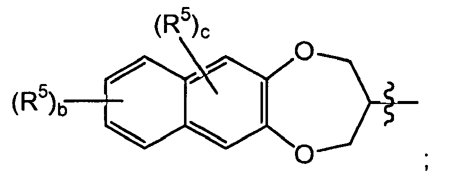 wherein b is an integer from 0 to 2; and wherein c is an integer from 0 to 1;
each R⁵ is independently selected from the group consisting of halogen, lower alkyl and nitro;
wherein "lower alkyl" refers to a carbon chain composition of 1-4 carbon atoms,
provided that when 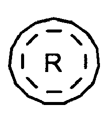 is 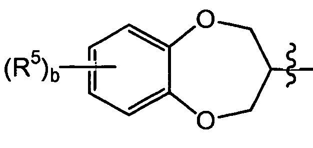 or 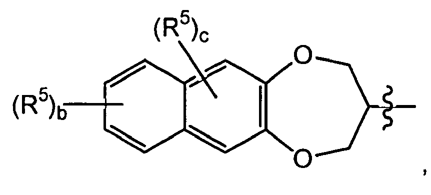 then a is 1.

3. The compound or a pharmaceutically acceptable salt thereof as in Claim 2 for use according to claim 2, wherein
R¹ and R² are each independently selected from the group consisting of hydrogen and lower alkyl;
R⁴ is selected from the group consisting of hydrogen and lower alkyl;
a is an integer from 1 to 2; 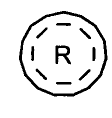 is selected from the group consisting of 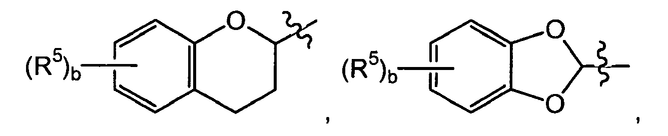 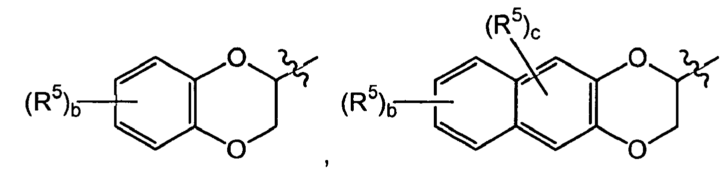 and 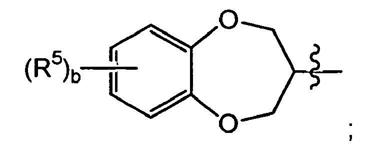 wherein b is an integer from 0 to 2; and wherein c is 0;
each R⁵ is independently selected from the group consisting of halogen, lower alkyl and nitro;
wherein "lower alkyl" refers to a carbon chain composition of 1-4 carbon atoms,
provided that when 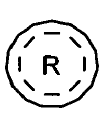 is 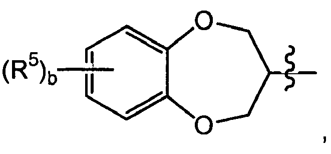 then a is 1.

4. The compound or a pharmaceutically acceptable salt thereof as in Claim 3 for use according to claim 3, wherein
R¹ and R² are each independently selected from the group consisting of hydrogen and lower alkyl;
R⁴ is selected from the group consisting of hydrogen and methyl; a is an integer from 1 to 2; 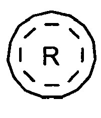 is selected from the group consisting of 2-(2,3-dihydrobenzo[1,4]dioxinyl), 2-(benzo[1,3]dioxolyl), 2-(3,4-dihydro-2H-benzo[1,4]dioxepinyl), 2-(2,3-dihydro-benzo[1,4]dioxinyl), 2-(6-chloro-2,3-dihydro-benzo[1,4]dioxinyl), 2-(6-fluoro-2,3-dihydro-benzo[1,4]dioxinyl), 2-(chromanyl), 2-(5-fluoro-2,3-dihydro-benzo[1,4]dioxinyl), 2-(7-chloro-2,3-dihydro-benzo[1,4]dioxinyl), 2-(6-chloro-benzo[1,3]dioxolyl), 2-(7-nitro-2,3-dihydro-benzo[1,4]dioxinyl), 2-(7-methyl-2,3-dihydro-benzo[1,4]dioxinyl), 2-(5-chloro-2,3-dihydro-benzo[1,4]dioxinyl), 2-(6-bromo-2,3-dihydrobenzo[1,4]dioxinyl), 2-(6,7-dichloro-2,3-dihydro-benzo[1,4]dioxinyl), 2-(8-chloro-2,3-dihydro-benzo[1,4]dioxinyl), 2-(2,3-dihydro-naphtho[2,3-b][1,4]dioxinyl) and 2-(4-methyl-benzo[1,3]dioxolyl);
wherein "lower alkyl" refers to a carbon chain composition of 1-4 carbon atoms,
provided that when 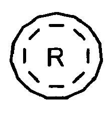 is 2-(3,4-dihydro-2H-benzo[1,4]dioxepinyl), then a is 1.

5. The compound or a pharmaceutically acceptable salt thereof as in Claim 4 for use according to claim 4, wherein
R¹ and R² are each independently selected from the group consisting of hydrogen and methyl;
R⁴ is selected from the group consisting of hydrogen and methyl;
a is an integer from 1 to 2; 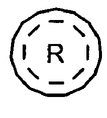 is selected from the group consisting of 2-(benzo[1,3]dioxolyl), 2-(2,3-dihydro-benzo[1,4]dioxinyl), 2-(2,3-dihydro-benzo[1,4]dioxinyl), 2-(6-chloro-2,3-dihydro-benzo[1,4]dioxinyl), 2-(7-chloro-2,3-dihydro-benzo[1,4]dioxinyl), 2-(7-methyl-2,3-dihydro-benzo[1,4]dioxinyl), 2-(6-bromo-2,3-dihydrobenzo[1,4]dioxinyl) and 2-(6,7-dichloro-2,3-dihydro-benzo[1,4]dioxinyl).

6. The compound or a pharmaceutically acceptable salt thereof of Claim 1 for use according to claim 1, wherein the compound of formula (I) is selected from the group consisting of (2S)-(-)-N-(6-chloro-2,3-dihydro-benzo[1,4]dioxin-2-ylmethyl)-sulfamide.

7. A compound selected from the group consisting of (2S)-(-)-N-(6-chloro-2,3-dihydro-benzo[1,4]dioxin-2-ylmethyl)-sulfamide; and pharmaceutically acceptable salts thereof for use in the treatment, prevention, arrest, inhibition or reversal of epileptogenesis, wherein said treatment comprises administering said compound or pharmaceutically acceptable salt to a patient in need of treatment with an anti-epileptogenic drug (an AEGD), wherein said patient is at risk for the development of epilepsy, a seizure disorder or an analogous seizure-related disorder.

8. The compound or pharmaceutically acceptable salt thereof for use according to any of Claims 1 and 7, wherein the predisposing factor(s) rendering the patient in need of treatment with an anti-epileptogenic drug (an AEGD) are selected from the group consisting of: injury or trauma of any kind to the CNS; neurosurgical procedures, activities that risk CNS injury, e.g., combat activities, auto or horse racing and contact sports including boxing; spinal cord trauma; infections of the CNS; anoxia; stroke (CVAs); history of Transient Ischemic Attacks (TIA's); carotid stenosis; history of athererosclerotic vessel disease; history of pulmonary emboli; peripheral vascular disease; autoimmune diseases affecting the CNS, e.g., lupus; birth injures, e.g., perinatal asphyxia; cardiac arrest; therapeutic or diagnostic vascular surgical procedures, e.g., carotid endarterectomy or cerebral angiography; hypotension; injury to the CNS from emboli, hyper or hypo perfusion; hypoxia; known genetic predisposition to disorders known to respond to AEGDs; space occupying lesions of the CNS; brain tumors, e.g., glioblastomas; bleeding or hemorrhage in or surrounding the CNS, e.g., intracerebral bleeds or subdural hematomas; brain edema; febrile convulsions; hyperthermia; exposure to toxic or poisonous agents; drug intoxication or withdrawal, e.g. cocaine, methamphetamine or alcohol; family history of; seizure disorders or an epilepsy related seizure like neurological disorder or seizure related disorder, history of status epilepticus; current treatment with medications that lower seizure threshold, e.g., lithium carbonate, thorazine or clozapine; evidence from surrogate markers or biomarkers that the patient is in need of treatment with an anti-epileptogenic drug, e.g. MRI scan showing hippocampal sclerosis, elevated serum levels of neuronal degradation products, elevated levels of ciliary neurotrophic factor (CNTF) or an EEG suggestive of a seizure disorder or an epilepsy related seizure like neurological disorder or an analogous seizure related disorder.

9. The compound or pharmaceutically acceptable salt thereof for use according to any of Claims 1 and 7, wherein the predisposing factor(s) rendering the patient in need of treatment with an anti-epileptogenic drug (an AEGD) are selected from the group consisting of: closed or penetrating head trauma; neurosurgical procedures, carotid stenosis, stroke or other cerebral-vascular accident (CVA); status epilepticus and space occupying lesions of the CNS.

10. The compound or pharmaceutically acceptable salt thereof for use according to any of Claims 1 and 7, wherein the said predisposing factor(s) are closed head trauma or penetrating head trauma or a neurosurgical procedure.

11. The compound or pharmaceutically acceptable salt thereof for use according to any of Claims 1 and 7, wherein the said predisposing factor(s) are; stroke, other cerebral-vascular accident (CVA), presence of carotid stenosis or Transient Ischemic Attack's.

12. The compound or pharmaceutically acceptable salt thereof for use according to any of Claims 1 and 7, wherein the said predisposing factor is status epilepticus.

13. A compound of formula (II) 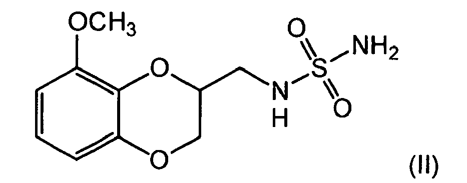 or a pharmaceutically acceptable salt thereof for use in the treatment, prevention, arrest, inhibition or reversal of epileptogenesis, wherein said compound or pharmaceutically acceptable salt thereof is administered to a patient in need of treatment with an anti-epileptogenic drug (an AEGD), wherein said patient is at risk for the development of epilepsy, a seizure disorder or an analogous seizure-related disorder.

## Patentansprüche

1. Verbindung der Formel (I) 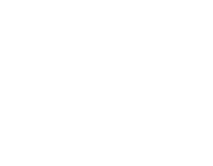 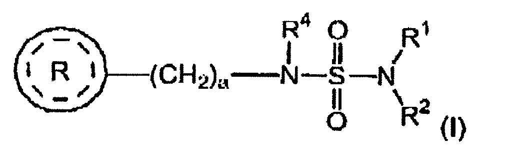 oder ein pharmazeutisch annehmbares Salz davon zur Verwendung bei der Behandlung, Prävention, Aufhaltung, Inhibierung oder Umkehr von Epileptogenese, wobei die Behandlung die Verabreichung der Verbindung bzw. des pharmazeutisch annehmbaren Salzes davon an einen Patienten, der einer Behandlung mit einem antiepileptogenen Arzneistoff (einem AEGD) bedarf, umfasst, wobei bei dem Patienten das Risiko der Entwicklung von Epilepsie, eines Anfallsleidens oder eines analogen mit Anfällen in Zusammenhang stehenden Leidens besteht,
wobei:
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht; 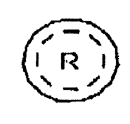 aus der Gruppe bestehend aus 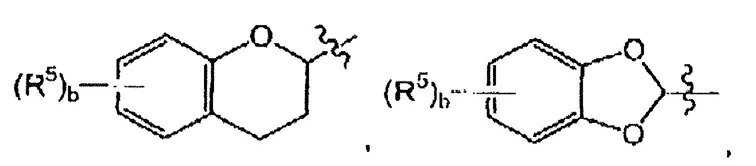 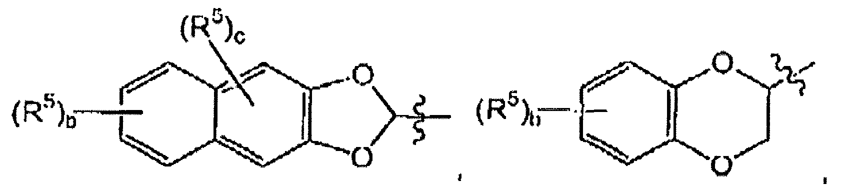 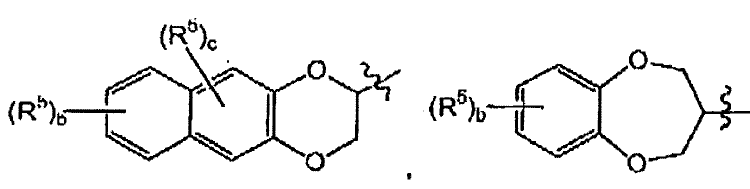 und 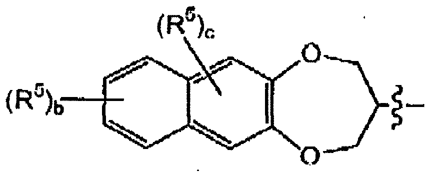
ausgewählt ist;
wobei b für eine ganze Zahl von 0 bis 4 steht und c für eine ganze Zahl von 0 bis 2 steht;
R⁵ jeweils unabhängig voneinander aus der Gruppe bestehend aus Halogen, Niederalkyl und Nitro ausgewählt ist;
wobei "Niederalkyl" sich auf eine Kohlenstoffkettenzusammensetzung mit 1-4 Kohlenstoffatomen bezieht,
mit der Maßgabe, dass dann, wenn 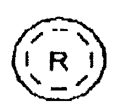 für 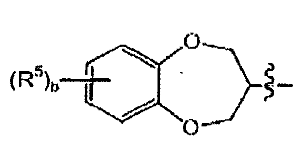 oder 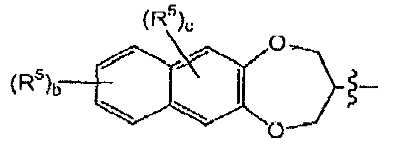 steht, a für 1 steht.

2. Verbindung oder pharmazeutisch annehmbares Salz davon nach Anspruch 1 zur Verwendung gemäß Anspruch 1, wobei:
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht; 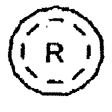 aus der Gruppe bestehend aus 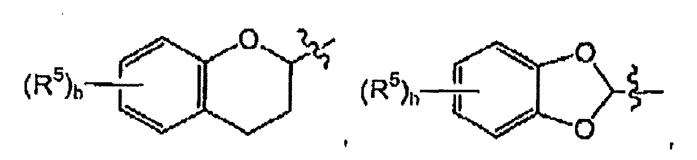 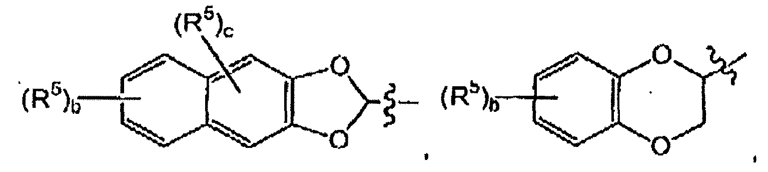 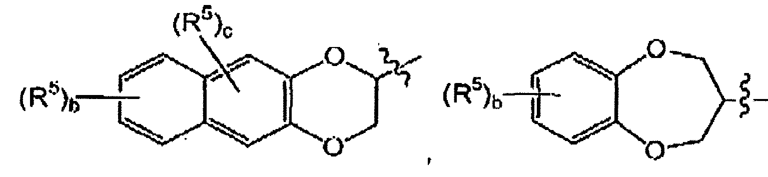 und 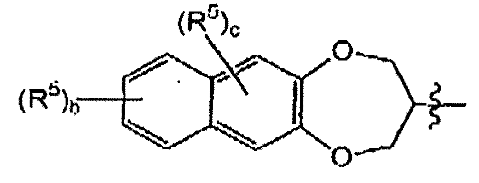
ausgewählt ist;
wobei b für eine ganze Zahl von 0 bis 2 steht und c für eine ganze Zahl von 0 bis 1 steht;
R⁵ jeweils unabhängig voneinander aus der Gruppe bestehend aus Halogen, Niederalkyl und Nitro ausgewählt ist;
wobei "Niederalkyl" sich auf eine Kohlenstoffkettenzusammensetzung mit 1-4 Kohlenstoffatomen bezieht,
mit der Maßgabe, dass dann, wenn 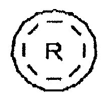 für 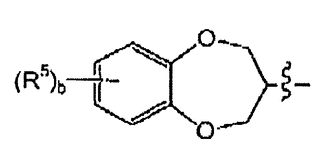 oder 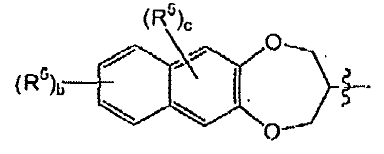 steht, a für 1 steht.

3. Verbindung oder pharmazeutisch annehmbares Salz davon nach Anspruch 2 zur Verwendung gemäß Anspruch 2, wobei:
R¹ und R² jeweils unabhängig voneinander aus der Gruppe bestehend aus Wasserstoff und Niederalkyl ausgewählt sind;
R⁴ aus der Gruppe bestehend aus Wasserstoff und Niederalkyl ausgewählt ist;
a für eine ganze Zahl von 1 bis 2 steht; 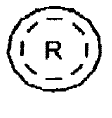 aus der Gruppe bestehend aus 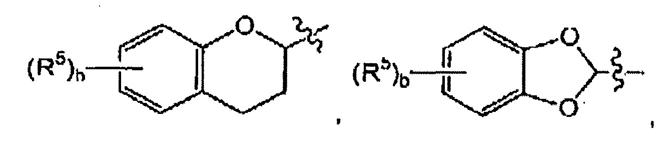 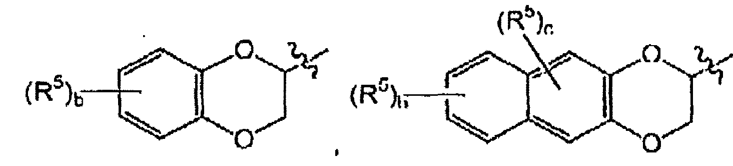 und 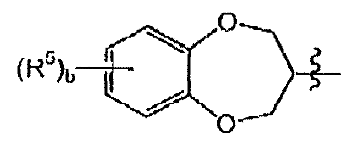
ausgewählt ist;
wobei b für eine ganze Zahl von 0 bis 2 steht und c für 0 steht;
R⁵ jeweils unabhängig voneinander aus der Gruppe bestehend aus Halogen, Niederalkyl und Nitro ausgewählt ist;
wobei "Niederalkyl" sich auf eine Kohlenstoffkettenzusammensetzung mit 1-4 Kohlenstoffatomen bezieht,
mit der Maßgabe, dass dann, wenn 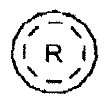 für 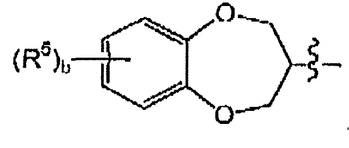 steht, a für 1 steht.

4. Verbindung oder pharmazeutisch annehmbares Salz davon nach Anspruch 3 zur Verwendung gemäß Anspruch 3, wobei:
R¹ und R² jeweils unabhängig voneinander aus der Gruppe bestehend aus Wasserstoff und Niederalkyl ausgewählt sind;
R⁴ aus der Gruppe bestehend aus Wasserstoff und Methyl ausgewählt ist;
a für eine ganze Zahl von 1 bis 2 steht; 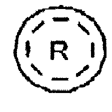 aus der Gruppe bestehend aus 2-(2,3-Dihydrobenzo[1,4]dioxinyl), 2-(Benzo[1,3]dioxolyl), 2-(3,4-Dihydro-2H-benzo[1,4]dioxepinyl), 2-(2,3-Dihydrobenzo[1,4]dioxinyl), 2-(6-Chlor-2,3-dihydrobenzo[1,4]dioxinyl), 2-(6-Fluor-2,3-dihydrobenzo[1,4]dioxinyl), 2-(Chromanyl), 2-(5-Fluor-2,3-dihydrobenzo[1,4]dioxinyl), 2-(7-Chlor-2,3-dihydrobenzo[1,4]dioxinyl), 2-(6-Chlorbenzo[1,3]-dioxolyl, 2-(7-Nitro-2,3-dihydrobenzo[1,4]-dioxinyl), 2-(7-Methyl-2,3-dihydrobenzo[1,4]-dioxinyl), 2-(5-Chlor-2,3-dihydrobenzo[1,4]-dioxinyl), 2-(6-Brom-2,3-dihydrobenzo[1,4]-dioxinyl), 2-(6,7-Dichlor-2,3-dihydrobenzo[1,4]-dioxinyl), 2-(8-Chlor-2,3-dihydrobenzo[1,4]-dioxinyl), 2-(2,3-Dihydronaphtho[2,3-b][1,4]-dioxinyl) und 2-(4-Methylbenzo[1,3]dioxolyl) ausgewählt ist;
wobei "Niederalkyl" sich auf eine Kohlenstoffkettenzusammensetzung mit 1-4 Kohlenstoffatomen bezieht,
mit der Maßgabe, dass dann, wenn 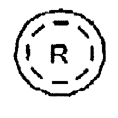 für 2-(3,4-Dihydro-2H-benzo[1,4]dioxepinyl) steht, a für 1 steht.

5. Verbindung oder pharmazeutisch annehmbares Salz davon nach Anspruch 4 zur Verwendung gemäß Anspruch 4, wobei:
R¹ und R² jeweils unabhängig voneinander aus der Gruppe bestehend aus Wasserstoff und Methyl ausgewählt sind;
R⁴ aus der Gruppe bestehend aus Wasserstoff und Methyl ausgewählt ist;
a für eine ganze Zahl von 1 bis 2 steht; 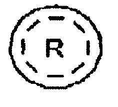 aus der Gruppe bestehend aus 2-(Benzo[1,3]dioxolyl), 2-(2,3-Dihydrobenzo[1,4]-dioxinyl), 2-(2,3-Dihydrobenzo[1,4]dioxinyl), 2-(6-Chlor-2,3-dihydrobenzo[1,4]dioxinyl), 2-(7-Chlor-2,3-dihydrobenzo[1,4]dioxinyl), 2-(7-Methyl-2,3-dihydrobenzo[1,4]dioxinyl), 2-(6-Brom-2,3-dihydrobenzo[1,4]dioxinyl) und 2-(6,7-Dichlor-2,3-dihydrobenzo[1,4]dioxinyl) ausgewählt ist.

6. Verbindung oder pharmazeutisch annehmbares Salz davon nach Anspruch 1 zur Verwendung gemäß Anspruch 1, wobei die Verbindung der Formel (I) aus der Gruppe bestehend aus (2S)-(-)-N-(6-Chlor-2,3-dihydrobenzo[1,4]dioxin-2-ylmethyl)sulfamid ausgewählt ist.

7. Verbindung aus der Gruppe bestehend aus (2S)-(-)-N-(6-Chlor-2,3-dihydrobenzo[1,4]dioxin-2-yl-methyl)sulfamid und pharmazeutisch annehmbare Salze davon zur Verwendung bei der Behandlung, Prävention, Aufhaltung, Inhibierung oder Umkehr von Epileptogenese, wobei die Behandlung die Verabreichung der Verbindung bzw. des pharmazeutisch annehmbaren Salzes an einen Patienten, der einer Behandlung mit einem antiepileptogenen Arzneistoff (einem AEGD) bedarf, umfasst, wobei bei dem Patienten das Risiko der Entwicklung von Epilepsie, eines Anfallsleidens oder eines analogen mit Anfällen in Zusammenhang stehenden Leidens besteht.

8. Verbindung oder pharmazeutisch annehmbares Salz davon zur Verwendung gemäß einem der Ansprüche 1 und 7, wobei der prädisponierende Faktor bzw. die prädisponierenden Faktoren, wegen dem bzw. wegen denen der Patient einer Behandlung mit einem antiepileptogenen Arzneistoff (einem AEGD) bedarf, aus der Gruppe bestehend aus ZNS-Verletzung oder -Trauma jeglicher Art; neurochirurgischen Eingriffen, Handlungen, bei denen das Risiko einer ZNS-Verletzung besteht, z.B. Kampfhandlungen, Auto- oder Pferderennen und Kontaktsportarten einschließlich Boxen; Rückenmarkstrauma; Infektionen des ZNS; Anoxie; Schlaganfall (CVA); Vorgeschichte von transitorischen ischämischen Attacken (ITA); Karotisstenose; Vorgeschichte von atherosklerotischer Gefäßerkrankung; Vorgeschichte von Lungenemboli; peripherer Verschlusskrankheit; Autoimmunerkrankungen, die das ZNS beeinflussen, z.B. Lupus; Geburtsverletzungen, z.B. perinataler Asphyxie; Herzstillstand; therapeutischen oder diagnostischen vaskulären chirurgischen Eingriffen, z.B. Karotisendarteriektomie oder Hirnangiopathie; Hypotonie; emboli-, hypertonie- oder hypotoniebedingter ZNS-Verletzung; Hypoxie; bekannter genetischer Prädisposition für Leiden, die bekanntlich auf AEGD ansprechen; raumfordernden Läsionen des ZNS; Hirntumoren, z.B. Glioblastomen; Blutung oder Hämorrhagie im ZNS oder um das ZNS, z.B. intrakraniellen Blutungen oder subduralen Hämatomen; Hirnödem; Fieberkrämpfen; Hyperthermie; Exposition gegenüber toxischen oder giftigen Agenzien; Drogenintoxikation oder -entzug, z.B. Kokain, Methamphetamin oder Alkohol; Familienvorgeschichte von Anfallsleiden oder einem mit Epilepsie in Zusammenhang stehenden Anfall wie neurologischem Leiden oder mit Anfällen in Zusammenhang stehenden Leiden, Vorgeschichte von Status epilepticus; gegenwärtiger Behandlung mit Medikationen, die die Anfallsschwelle herabsetzen, z.B. Lithiumcarbonat, Thorazin oder Clozapin; von Surrogat-Markern oder -Biomarkern herrührenden Anzeichen, dass der Patient einer Behandlung mit einem antiepileptogenen Arzneistoff bedarf, z.B. einem MRI-Scan, der Hippocampussklerose zeigt, erhöhten Serumspiegeln von neuronalen Abbauprodukten, erhöhten Spiegeln von CNTF (Ciliary Neutrotrophic Factor) oder einem EEG, das auf ein Anfallsleiden oder einen mit Epilepsie in Zusammensetzung stehenden Anfall wie ein neurologisches Leiden oder ein analoges mit Anfällen in Zusammenhang stehendes Leiden hindeutet; ausgewählt ist bzw. sind.

9. Verbindung oder pharmazeutisch annehmbares Salz davon zur Verwendung gemäß einem der Ansprüche 1 und 7, wobei der prädisponierende Faktor bzw. die prädisponierenden Faktoren, wegen dem bzw. wegen denen der Patient einer Behandlung mit einem antiepileptogenen Arzneistoff (einem AEGD) bedarf, aus der Gruppe bestehend aus geschlossenem oder penetrierendem Kopftrauma; neurochirurgischen Eingriffen, Karotisstenose, Schlaganfall oder einem anderen CVA (Cerebral-Vascular Accident); Status epilepticus und raumfordernden Läsionen des ZNS ausgewählt ist bzw. sind.

10. Verbindung oder pharmazeutisch annehmbares Salz davon zur Verwendung gemäß einem der Ansprüche 1 und 7, wobei es sich bei dem prädisponierenden Faktor bzw. den prädisponierenden Faktoren um geschlossenes Kopftrauma oder penetrierendes Kopftrauma oder einen neurochirurgischen Eingriff handelt.

11. Verbindung oder pharmazeutisch annehmbares Salz davon zur Verwendung gemäß einem der Ansprüche 1 und 7, wobei es sich bei dem prädisponierenden Faktor bzw. den prädisponierenden Faktoren um Schlaganfall, einen anderen CVA (Cerebral-Vascular Accident), Vorliegen von Karotisstenose oder transitorische ischämische Attacken handelt.

12. Verbindung oder pharmazeutisch annehmbares Salz davon zur Verwendung gemäß einem der Ansprüche 1 und 7, wobei es sich bei dem prädisponierenden Faktor um Status epilepticus handelt.

13. Verbindung der Formel (II) 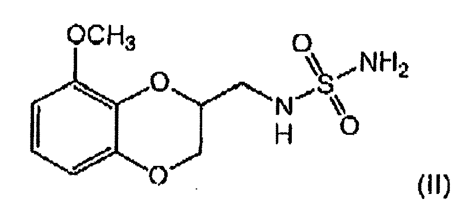 oder ein pharmazeutisch annehmbares Salz davon zur Verwendung bei der Behandlung, Prävention, Aufhaltung, Inhibierung oder Umkehr von Epileptogenese, wobei die Verbindung bzw. das pharmazeutisch annehmbare Salz davon an einen Patienten, der einer Behandlung mit einem antiepileptogenen Arzneistoff (einem AEGD) bedarf, verabreicht wird, wobei bei dem Patienten das Risiko der Entwicklung von Epilepsie, eines Anfallsleidens oder eines analogen mit Anfällen in Zusammenhang stehenden Leidens besteht.

## Revendications

1. Composé de formule (I) 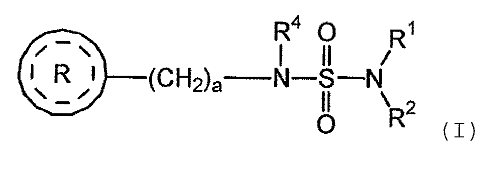 ou un sel pharmaceutiquement acceptable de celui-ci pour utilisation dans le traitement, la prévention, l'arrêt, l'inhibition ou l'inversion de l'épileptogenèse, ledit traitement comprenant l'administration dudit composé ou d'un sel pharmaceutiquement acceptable de celui-ci à un patient nécessitant un traitement avec un médicament antiépileptogenèse (un AEGD), ledit patient présentant un risque de développement d'épilepsie, d'un trouble convulsif ou d'un trouble de type convulsif analogue, dans lequel
R¹ et R² sont chacun indépendamment choisis dans le groupe constitué d'hydrogène et alkyle inférieur ;
R⁴ est choisi dans le groupe constitué d'hydrogène et alkyle inférieur ;
a est un entier de 1 à 2 ; 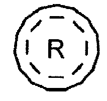 est choisi dans le groupe constitué de 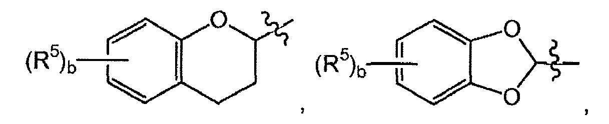 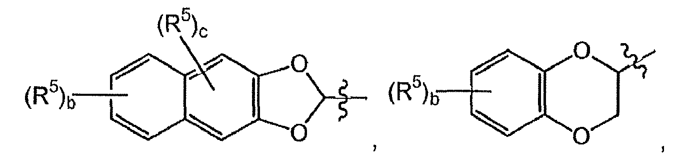 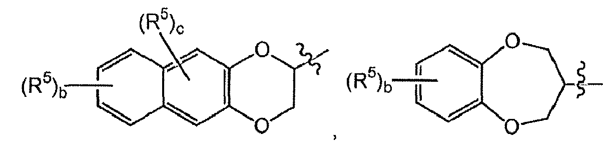 et 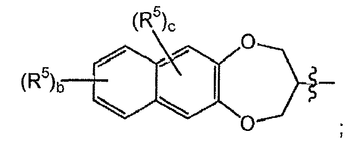 où b est un entier de 0 à 4 ; et où c est un entier de 0 à 2 ;
chaque R⁵ est indépendamment choisi dans le groupe constitué d'halogène, alkyle inférieur et nitro ;
où « alkyle inférieur » désigne une composition de chaîne de carbone de 1 à 4 atomes de carbone,
à condition que lorsque 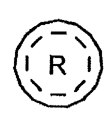 est 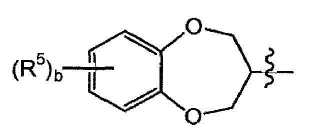 ou 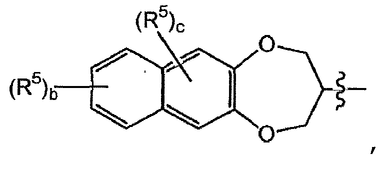 alors a est 1.

2. Composé ou un sel pharmaceutiquement acceptable de celui-ci selon la revendication 1 pour utilisation selon la revendication 1, dans lequel
R¹ et R² sont chacun indépendamment choisis dans le groupe constitué d'hydrogène et alkyle inférieur ;
R⁴ est choisi dans le groupe constitué d'hydrogène et alkyle inférieur ;
a est un entier de 1 à 2 ; 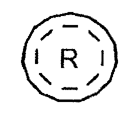 est choisi dans le groupe constitué de 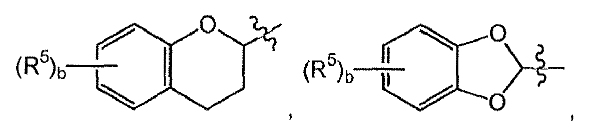 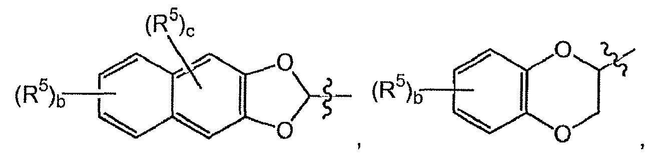 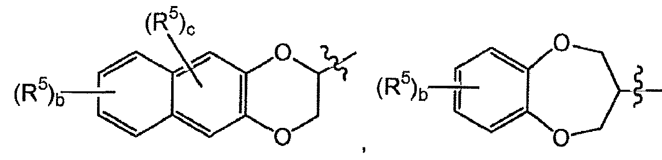 et 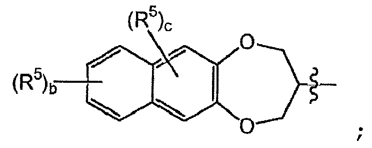 où b est un entier de 0 à 2 ; et où c est un entier de 0 à 1 ;
chaque R⁵ est indépendamment choisi dans le groupe constitué d'halogène, alkyle inférieur et nitro ;
où « alkyle inférieur » désigne une composition de chaîne de carbone de 1 à 4 atomes de carbone,
à condition que lorsque 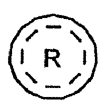 est 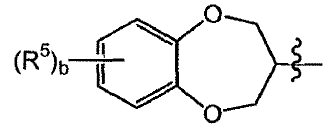 ou 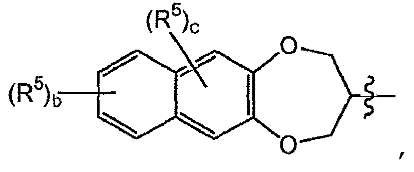 alors a est 1.

3. Composé ou sel pharmaceutiquement acceptable de celui-ci selon la revendication 2 pour utilisation selon la revendication 2, où
R¹ et R² sont chacun indépendamment choisis dans le groupe constitué d'hydrogène et alkyle inférieur ;
R⁴ est choisi dans le groupe constitué d'hydrogène et alkyle inférieur ;
a est un entier de 1 à 2 ; 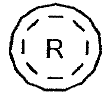 est choisi dans le groupe constitué de 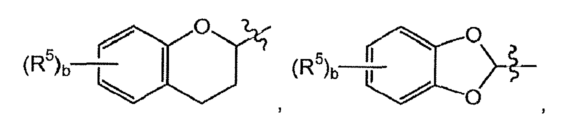 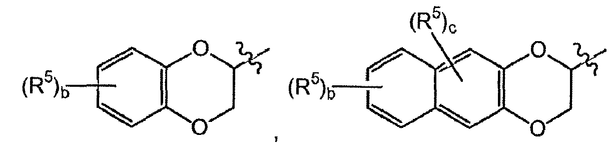 et 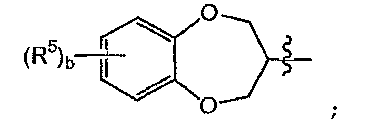 où b est un entier de 0 à 2 ; et où c est 0 ;
chaque R⁵ est indépendamment choisi dans le groupe constitué d'halogène, alkyle inférieur et nitro ;
où « alkyle inférieur » désigne une composition de chaîne de carbone de 1 à 4 atomes de carbone,
à condition que lorsque 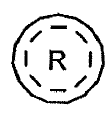 est 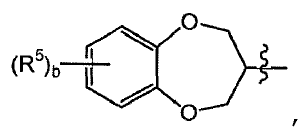 alors a est 1.

4. Composé ou un sel pharmaceutiquement acceptable de celui-ci selon la revendication 3 pour utilisation selon la revendication 3, où
R¹ et R² sont chacun indépendamment choisis dans le groupe constitué d'hydrogène et alkyle inférieur ;
R⁴ est choisi dans le groupe constitué d'hydrogène et méthyle ;
a est un entier de 1 à 2 ; 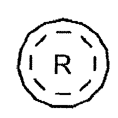 est choisi dans le groupe constitué de 2-(2,3-dihydro-benzo[1,4]dioxinyle), 2-(benzo[1,3]dioxolyle), 2-(3,4-dihydro-2H-benzo[1,4]dioxépinyle), 2-(2,3-dihydro-benzo[1,4]dioxinyle), 2-(6-chloro-2,3-dihydrobenzo[1,4]dioxinyle), 2-(6-fluoro-2,3-dihydrobenzo[1,4]dioxinyle), 2-(chromanyle), 2-(5-fluoro-2,3-dihydro-benzo[1,4]dioxinyle), 2-(7-chloro-2,3-dihydrobenzo[1,4]dioxinyle), 2-(6-chloro-benzo[1,3]dioxolyle), 2-(7-nitro-2,3-dihydro-benzo[1,4]dioxinyle), 2-(7-méthyl-2,3-dihydro-benzo[1,4]dioxinyle), 2-(5-chloro-2,3-dihydro-benzo[1,4]dioxinyle), 2-(6-bromo-2,3-dihydro-benzo[1,4]dioxinyle), 2-(6,7-dichloro-2,3-dihydro-benzo[1,4]dioxinyle), 2-(8-chloro-2,3-dihydrobenzo[1,4]dioxinyle), 2-(2,3-dihydro-naphto[2,3-b][1,4]dioxinyle) et 2-(4-méthyl-benzo[1,3]dioxolyle) ; « alkyle inférieur » désignant une composition de chaîne de carbone de 1 à 4 atomes de carbone,
à condition que lorsque 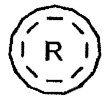 est 2-(3,4-dihydro-2H-benzo[1,4]dioxépinyle), alors a est 1.

5. Composé ou un sel pharmaceutiquement acceptable de celui-ci selon la revendication 4 pour utilisation selon la revendication 4, dans lequel
R¹ et R² sont chacun indépendamment choisis dans le groupe constitué d'hydrogène et méthyle ;
R⁴ est choisi dans le groupe constitué d'hydrogène et méthyle ;
a est un entier de 1 à 2 ; 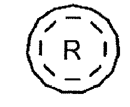 est choisi dans le groupe constitué de 2-(benzo[1,3]dioxolyle), 2-(2,3-dihydrobenzo[1,4]dioxinyle), 2-(2,3-dihydrobenzo[1,4]dioxinyle), 2-(6-chloro-2,3-dihydrobenzo[1,4]dioxinyle), 2-(7-chloro-2,3-dihydrobenzo[1,4]dioxinyle), 2-(7-méthyl-2,3-dihydrobenzo[1,4]dioxinyle), 2-(6-bromo-2,3-dihydrobenzo[1,4]dioxinyle) et 2-(6,7-dichloro-2,3-dihydrobenzo[1,4]dioxinyle).

6. Composé ou un sel pharmaceutiquement acceptable de celui-ci de la revendication 1 pour utilisation selon la revendication 1, où le composé de formule (I) est choisi dans le groupe constitué de (2S)-(-)-N-(6-chloro-2,3-dihydro-benzo[1,4]dioxin-2-ylméthyl)-sulfamide.

7. Composé choisi dans le groupe constitué de (2S)-(-)-N-(6-chloro-2,3-dihydro-benzo[1,4]dioxin-2-ylméthyl)-sulfamide ; et des sels pharmaceutiquement acceptables de celui-ci pour utilisation dans le traitement, la prévention, l'arrêt, l'inhibition ou l'inversion de l'épileptogenèse, ledit traitement comprenant l'administration dudit composé ou sel pharmaceutiquement acceptable à un patient nécessitant un traitement avec un médicament antiépileptogenèse (un AEGD), ledit patient présentant un risque de développement d'épilepsie, d'un trouble convulsif ou d'un trouble de type convulsif analogue.

8. Composé ou sel pharmaceutiquement acceptable de celui-ci pour utilisation selon l'une quelconque des revendications 1 et 7, le(s) facteur(s) prédisposant(s) amenant le patient à nécessiter un traitement avec un médicament antiépileptogenèse (un AEGD) étant choisis dans le groupe constitué de : lésion ou traumatisme de type quelconque du SNC ; procédures neurochirurgicales, activités présentant un risque de lésion du SNC, par exemple, des activités de combat, courses automobiles ou de chevaux et sports de contact comprenant la boxe ; traumatisme de la moelle épinière ; infections du SNC ; anoxie ; accident vasculaire cérébral (AVC) ; antécédent d'attaques ischémiques transitoires (AIT) ; sténose carotidienne ; antécédent de maladie vasculaire athérosclérotique ; antécédent d'embolie pulmonaire ; maladie vasculaire périphérique ; maladies auto-immunes affectant le SNC, par exemple, le lupus ; lésions périnatales, par exemple, asphyxie périnatale ; arrêt cardiaque ; procédures chirurgicales vasculaires thérapeutiques ou diagnostiques, par exemple, l'endartérectomie carotidienne ou l'angiographie cérébrale ; hypotension ; lésion du SNC consécutive à une embolie, une hyper- ou une hypoperfusion ; hypoxie ; prédisposition génétique connue à des troubles connus pour répondre à des AEGD ; lésions du SNC occupant l'espace ; tumeurs cérébrales, par exemple, des glioblastomes ; saignement ou hémorragie dans ou autour du SNC, par exemple, des saignements intracérébraux ou des hématomes sous-duraux ; oedème cérébral ; convulsions fébriles ; hyperthermie ; exposition à des agents toxiques ou venimeux ; intoxication ou sevrage associé à une substance, par exemple la cocaïne, la méthamphétamine ou l'alcool ; antécédents familiaux de troubles convulsifs ou d'un trouble neurologique convulsif de type épileptique ou trouble de type convulsif, antécédent d'état épileptique ; traitement actuel avec des médicaments qui abaissent le seuil convulsivant, par exemple, le carbonate de lithium, la thorazine ou la clozapine ; signes de marqueurs ou biomarqueurs de substitution indiquant que le patient nécessite un traitement avec un médicament antiépileptogenèse, par exemple, un scan IRM indiquant une sclérose hippocampique, des taux sériques élevés de produits de dégradation neuronaux, des taux élevés de facteur neurotrophique ciliaire (CNTF) ou un EEG suggérant un trouble convulsif ou un trouble neurologique convulsif de type épileptique ou un trouble de type convulsif analogue.

9. Composé ou sel pharmaceutiquement acceptable de celui-ci pour utilisation selon l'une quelconque des revendications 1 et 7, le(s) facteur(s) prédisposant(s) amenant le patient à nécessiter un traitement avec un médicament antiépileptogenèse (un AEGD) étant choisis dans le groupe constitué de : un traumatisme crânien fermé ou pénétrant ; des procédures neurochirurgicales, une sténose carotidienne, une attaque cérébrale ou un autre accident vasculaire cérébral (AVC) ; un état épileptique et des lésions du SNC occupant l'espace.

10. Composé ou sel pharmaceutiquement acceptable de celui-ci pour utilisation selon l'une quelconque des revendications 1 et 7, lesdits facteur(s) prédisposant(s) étant un traumatisme crânien fermé ou un traumatisme crânien pénétrant ou une procédure neurochirurgicale.

11. Composé ou sel pharmaceutiquement acceptable de celui-ci pour utilisation selon l'une quelconque des revendications 1 et 7, dans lequel lesdits facteur(s) prédisposant(s) sont : une attaque cérébrale, un autre accident vasculaire cérébral (AVC), la présence de sténose carotidienne ou une attaque ischémique transitoire.

12. Composé ou sel pharmaceutiquement acceptable de celui-ci pour utilisation selon l'une quelconque des revendications 1 et 7, ledit facteur prédisposant étant un état épileptique.

13. Composé de formule (II) 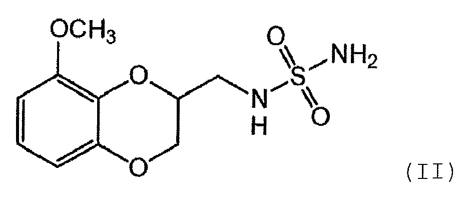 ou un sel pharmaceutiquement acceptable de celui-ci pour utilisation dans le traitement, la prévention, l'arrêt, l'inhibition ou l'inversion de l'épileptogenèse, ledit composé ou un sel pharmaceutiquement acceptable de celui-ci étant administré à un patient nécessitant un traitement avec un médicament antiépileptogenèse (un AEGD), ledit patient présentant un risque de développement d'épilepsie, de trouble convulsif ou de trouble de type convulsif analogue.
